# EUROPEAN PATENT SPECIFICATION

(11) **EP 3 404 735 B1**
(45) Date of publication and mention of the grant of the patent: **10.05.2023**
(21) Application number: 17738565.5
(22) Date of filing: 13.01.2017
(51) Int. Cl.: H01L 51/54, C07C 211/54, C07C 211/61, C07D 209/86, C09K 11/06

(54) **ORGANIC ELECTROLUMINESCENT DEVICE**
ORGANISCHES ELEKTROLUMINESZENTES ELEMENT
ÉLÉMENT ÉLECTROLUMINESCENT ORGANIQUE

(30) Priority: 14.01.2016 JP 2016004843
(43) Date of publication of application: 21.11.2018
(73) Proprietor: Hodogaya Chemical Co., Ltd., Chuo-ku, Tokyo 104-0028 (JP)
(72) Inventor: HAYASHI, Shuichi, Tokyo 104-0028 (JP); KABASAWA, Naoaki, Tokyo 104-0028 (JP); YAMAMOTO, Takeshi, Tokyo 104-0028 (JP); MOCHIZUKI, Shunji, Tokyo 104-0028 (JP)
(74) Representative: Müller-Boré & Partner Patentanwälte PartG mbB
(86) International application number: PCT/JP2017/001112
(87) International publication number: WO 2017/122813

(56) References cited:
- WO-A1-2007/043484
- WO-A1-2015/001726
- WO-A1-2015/041428
- CN-A- 105 566 123
- JP-A- 2002 356 462
- JP-A- 2002 356 462
- JP-A- 2004 253 298
- JP-A- 2013 080 961
- KR-A- 20110 084 798
- KR-A- 20110 084 798
- KR-A- 20150 007 476
- KR-A- 20150 130 206
- KR-A- 20150 130 206
- US-A1- 2005 236 976
- US-A1- 2005 236 976

## Description

### Technical Field

The present invention relates to an organic electroluminescent device which is a preferred self-luminous device for various display devices. Specifically, this invention relates to specific arylamine compounds, and organic electroluminescent devices (hereinafter referred to as organic EL devices) using specific arylamine compounds (and compounds having a pyrimidine ring structure having a particular structure).

### Background Art

The organic EL device is a self-luminous device and has been actively studied for their brighter, superior visibility and the ability to display clearer images in comparison with liquid crystal devices.

In 1987, C. W. Tang and colleagues at Eastman Kodak developed a laminated structure device using materials assigned with different roles, realizing practical applications of an organic EL device with organic materials. These researchers laminated an electron-transporting phosphor and a hole-transporting organic substance, and injected both charges into a phosphor layer to cause emission in order to obtain a high luminance of 1,000 cd/m² or more at a voltage of 10 V or less (refer to PTLs 1 and 2, for example).

To date, various improvements have been made for practical applications of the organic EL device. Various roles of the laminated structure are further subdivided to provide an electroluminescence device that includes an anode, a hole injection layer, a hole transport layer, a light emitting layer, an electron transport layer, an electron injection layer, and a cathode successively formed on a substrate, and high efficiency and durability have been achieved by the electroluminescence device (refer to NPL 1, for example).

Further, there have been attempts to use triplet excitons for further improvements of luminous efficiency, and the use of a phosphorescence-emitting compound has been examined (refer to NPL 2, for example).

Devices that use light emission caused by thermally activated delayed fluorescence (TADF) have also been developed. In 2011, Adachi et al. at Kyushu University, National University Corporation realized 5.3% external quantum efficiency with a device using a thermally activated delayed fluorescent material (refer to NPL 3, for example).

The light emitting layer can be also fabricated by doping a charge-transporting compound generally called a host material, with a fluorescent compound, a phosphorescence-emitting compound, or a delayed fluorescent-emitting material. As described in the NPL, the selection of organic materials in an organic EL device greatly influences various device characteristics such as efficiency and durability (refer to NPL 2, for example).

In an organic EL device, charges injected from both electrodes recombine in a light emitting layer to cause emission. What is important here is how efficiently the hole and electron charges are transferred to the light emitting layer in order to form a device having excellent carrier balance. The probability of hole-electron recombination can be improved by improving hole injectability and electron blocking performance of blocking injected electrons from the cathode, and high luminous efficiency can be obtained by confining excitons generated in the light emitting layer. The role of a hole transport material is therefore important, and there is a need for a hole transport material that has high hole injectability, high hole mobility, high electron blocking performance, and high durability to electrons.

Heat resistance and amorphousness of the materials are also important with respect to the lifetime of the device. The materials with low heat resistance cause thermal decomposition even at a low temperature by heat generated during the drive of the device, which leads to the deterioration of the materials. The materials with low amorphousness cause crystallization of a thin film even in a short time and lead to the deterioration of the device. The materials in use are therefore required to have characteristics of high heat resistance and satisfactory amorphousness.

N,N'-diphenyl-N,N'-di(α-naphthyl)benzidine (NPD) and various aromatic amine derivatives are known as the hole transport materials used for the organic EL device (refer to PTLs 1 and 2, for example). Although NPD has desirable hole transportability, its glass transition point (Tg), which is an index of heat resistance, is as low as 96°C, which causes the degradation of device characteristics by crystallization under a high-temperature condition (refer to NPL 4, for example). The aromatic amine derivatives described in the PTLs include a compound known to have an excellent hole mobility of 10⁻³ cm²/Vs or higher (refer to PTLs 1 and 2, for example). However, since the compound is insufficient in terms of electron blocking performance, some of the electrons pass through the light emitting layer, and improvements in luminous efficiency cannot be expected. For such a reason, a material with higher electron blocking performance, a more stable thin-film state and higher heat resistance is needed for higher efficiency. Although an aromatic amine derivative having high durability is reported (refer to PTL 3, for example), the derivative is used as a charge transporting material used in an electrophotographic photoconductor, and there is no example of using the derivative in the organic EL device.

Arylamine compounds having a substituted carbazole structure are proposed as compounds improved in the characteristics such as heat resistance and hole injectability (refer to PTLs 4 and 5, for example). However, while the devices using these compounds for the hole injection layer or the hole transport layer have been improved in heat resistance, luminous efficiency and the like, the improvements are still insufficient. Further lower driving voltage and higher luminous efficiency are therefore needed.

In order to improve characteristics of the organic EL device and to improve the yield of the device production, it has been desired to develop a device having high luminous efficiency, low driving voltage and a long lifetime by using in combination the materials that excel in hole and electron injection/transport performances, stability as a thin film and durability, permitting holes and electrons to be highly efficiently recombined together.

Further, in order to improve characteristics of the organic EL device, it has been desired to develop a device that maintains carrier balance and has high efficiency, low driving voltage and a long lifetime by using in combination the materials that excel in hole and electron injection/transport performances, stability as a thin film and durability.

Documents KR20150007476 (A), WO2007/043484 (A), JP2002356462 (A), and WO2015/041428 (A) disclose triarylamine compounds for use on a hole transport layer of an OLED.

### Citation List

### Patent Literature

PTL 1: JP-A-8-048656
PTL 2: Japanese Patent No. 3194657
PTL 3: Japanese Patent No. 4943840
PTL 4: JP-A-2006-151979
PTL 5: WO2008/62636
PTL 6: KR-A-10-2015-0130206
PTL 7: KR-A-10-2013-0060157

### Non Patent Literature

NPL 1: The Japan Society of Applied Physics, 9th Lecture Preprints, pp. 55 to 61 (2001)
NPL 2: The Japan Society of Applied Physics, 9th Lecture Preprints, pp. 23 to 31 (2001)
NPL 3: Appl. Phys. Let., 98, 083302 (2011)
NPL 4: Organic EL Symposium, the 3rd Regular presentation Preprints, pp. 13 to 14 (2006)

### Summary of Invention

### Technical Problem

An object of the present invention is to provide a material for an organic EL device that is excellent in hole injection and transport abilities, electron blocking ability, thin film stability, and durability, as a material for an organic EL device with high efficiency and high durability, and also to provide an organic EL device having a high efficiency, a low driving voltage, and a long lifetime by combining the material with various materials for an organic EL device that is excellent in hole and electron injection and transport abilities, electron blocking ability, thin film stability, and durability, in such a manner that the characteristics of the materials can be effectively exhibited.

Physical properties of the organic compound to be provided by the present invention include (1) good hole injection characteristics, (2) large hole mobility, (3) stability in a thin-film state, and (4) excellent heat resistance. Physical properties of the organic EL device to be provided by the present invention include (1) high luminous efficiency and high power efficiency, (2) low turn on voltage, (3) low actual driving voltage, and (4) a long lifetime.

### Solution to Problem

For achieving the object, the present inventors have focused the fact that an arylamine material is excellent in hole injection ability and transport ability, thin film stability, and durability, and they have synthesized various compounds and have earnestly investigated the characteristics thereof. As a result, it has been found that an arylamine compound substituted with an aryl group at a particular position can efficiently inject and transport holes to a light emitting layer. Furthermore, they have focused the fact that a compound having a pyrimidine ring structure is excellent in electron injection ability and transport ability, thin film stability, and durability, and they have produced various organic EL devices in such a manner that the arylamine compound substituted with an aryl group at a particular position and a compound having a pyrimidine ring structure having a particular structure are selected to inject and transport holes and electrons efficiently to a light emitting layer including a specific light-emitting material(dopant), and the hole transport material having a particular structure, the specific light-emitting material(dopant), and the electron transport material are combined to maintain carrier balance, and have earnestly investigated the characteristics of the devices. As a result, they have completed the present invention.

Specifically, according to the present invention, the organic EL devices according to the claims are provided.

An organic EL device according to the claims comprises the hole transport layer comprising an arylamine compound of the following general formula (1): 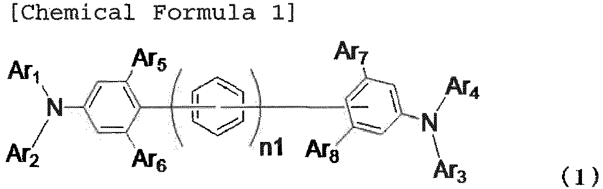 wherein Ar₁ to Ar₄ may be the same or different, and represent a substituted or unsubstituted aromatic hydrocarbon group, a substituted or unsubstituted aromatic heterocyclic group, or a substituted or unsubstituted condensed polycyclic aromatic group;Ar₅ represents a substituted or unsubstituted aromatic hydrocarbon group or a substituted or unsubstituted condensed polycyclic aromatic group; Ar₆ represents a hydrogen atom, a substituted or unsubstituted aromatic hydrocarbon group or a substituted or unsubstituted condensed polycyclic aromatic group, wherein the substituents in the substituted aromatic hydrocarbon group, or the substituted condensed polycyclic aromatic group of Ar₅ and Ar₆ are selected from the group consisting of a deuterium atom, cyano, nitro, halogen atoms, linear or branched alkyls of 1 to 6 carbon atoms, linear or branched alkyloxys of 1 to 6 carbon atoms, alkenyls, aryloxys, arylalkyloxys, aromatic hydrocarbon groups, condensed polycyclic aromatic groups, arylvinyls and acyls;Ar₇ and Ar₈ represent a hydrogen atom; and nl represents 0,1 or 2, where Ar₃ and Ar₄ may bind to each other to form a ring via a linking group, such as a single bond, substituted or unsubstituted methylene, an oxygen atom, a sulfur atom, or a monosubstituted amino group; and Ar₃ and Ar₄ may bind to the benzene ring binding with - NAr₃ Ar₄ group to form a ring via a linking group such as a single bond, substituted or unsubstituted methylene, an oxygen atom, a sulfur atom, or a monosubstituted amino group.

Preferably, the arylamine compound according to the claims is an arylamine compound of the following general formula (1a).

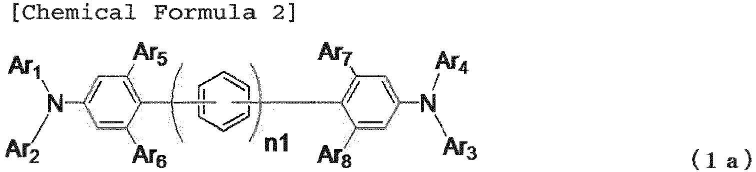

In the formula, Ar₁ to Ar₄ may be the same or different, and represent a substituted or unsubstituted aromatic hydrocarbon group, a substituted or unsubstituted aromatic heterocyclic group, or a substituted or unsubstituted condensed polycyclic aromatic group; Ar₅ represents a substituted or unsubstituted aromatic hydrocarbon group or a substituted or unsubstituted condensed polycyclic aromatic group; Ar₆ represents a hydrogen atom, a substituted or unsubstituted aromatic hydrocarbon group or a substituted or unsubstituted condensed polycyclic aromatic group, wherein the substituents in the substituted aromatic hydrocarbon group, or the substituted condensed polycyclic aromatic group of Ar₅ and Ar₆ are selected from the group consisting of a deuterium atom, cyano, nitro, halogen atoms, linear or branched alkyls of 1 to 6 carbon atoms, linear or branched alkyloxys of 1 to 6 carbon atoms, alkenyls, aryloxys, arylalkyloxys, aromatic hydrocarbon groups, condensed polycyclic aromatic groups, arylvinyls and acyls;Ar₇ and Ar₈ represent a hydrogen atom; and n1 represents 0,1 or 2, where Ar₃ and Ar₄ may bind to each other to form a ring via a linking group, such as a single bond, substituted or unsubstituted methylene, an oxygen atom, a sulfur atom, or a monosubstituted amino group; and Ar₃ and Ar₄ may bind to the benzene ring binding with -NAr₃ Ar₄ group to form a ring via a linking group such as a single bond, substituted or unsubstituted methylene, an oxygen atom, a sulfur atom, or a monosubstituted amino group.

The light emitting layer of the EL device according to the claims may comprise a blue light emitting dopant which is an amine derivative having a condensed ring structure of the following general formula (2) .

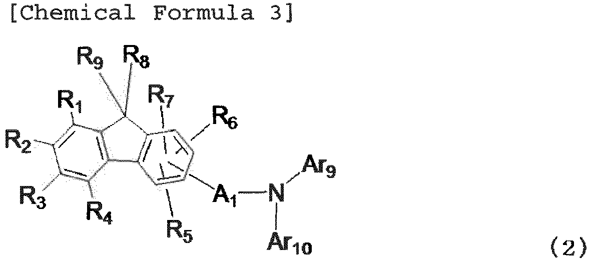

In the formula, A₁ represents a divalent group of a substituted or unsubstituted aromatic hydrocarbon, a divalent group of a substituted or unsubstituted aromatic heterocyclic ring, a divalent group of substituted or unsubstituted condensed polycyclic aromatics, or a single bond. Ar₉ and Ar₁₀ may be the same or different, and represent a substituted or unsubstituted aromatic hydrocarbon group, a substituted or unsubstituted aromatic heterocyclic group, or a substituted or unsubstituted condensed polycyclic aromatic group. Ar₉ and Ar₁₀ may bind to each other to form a ring via a linking group, such as a single bond, substituted or unsubstituted methylene, an oxygen atom, a sulfur atom, or a monosubstituted amino group. R₁ to R₄ may be the same or different, and represent a hydrogen atom, a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, substituted or unsubstituted aryloxy, or a disubstituted amino group substituted with a group selected from an aromatic hydrocarbon group, an aromatic heterocyclic group, and a condensed polycyclic aromatic group. These groups may bind to each other to form a ring via a linking group, such as a single bond, substituted or unsubstituted methylene, an oxygen atom, a sulfur atom, or a monosubstituted amino group. These groups bind to the benzene ring binding with R₁ to R₄ to form a ring via a linking group such as substituted or unsubstituted methylene, an oxygen atom, a sulfur atom, or a monosubstituted amino group. R₅ to R₇ may be the same or different, and represent a hydrogen atom, a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy. These groups may bind to each other to form a ring via a linking group, such as a single bond, substituted or unsubstituted methylene, an oxygen atom, a sulfur atom, or a monosubstituted amino group. These groups may bind to the benzene ring binding with R₅ to R₇ to form a ring via a linking group such as substituted or unsubstituted methylene, an oxygen atom, a sulfur atom, or a monosubstituted amino group. R₈ and R₉ may be the same or different, and represent linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy. These groups may bind to each other to form a ring via a linking group, such as a single bond, substituted or unsubstituted methylene, an oxygen atom, a sulfur atom, or a monosubstituted amino group.

The electron transport layer of the EL device according to the claims includes a compound of the following general formula (3) having a pyrimidine ring structure. 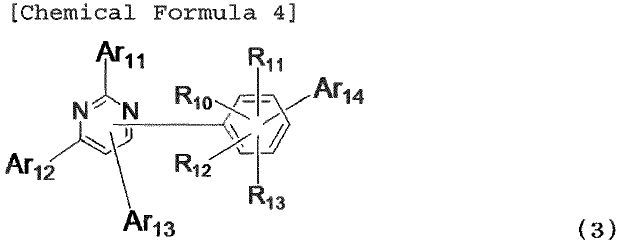 wherein Ar₁₁ represents a substituted or unsubstituted aromatic hydrocarbon group, or a substituted or unsubstituted condensed polycyclic aromatic group, wherein the substituents in the substituted aromatic hydrocarbon group, or the substituted condensed polycyclic aromatic group of Ar₁₁ are selected from the group consisting of a deuterium atom, cyano, nitro, a fluorine atom, a chlorine atom, a bromine atom, an iodine atom, methyl, ethyl, n-propyl, isopropyl, n-butyl, isobutyl, tert-butyl, n-pentyl, isopentyl, neopentyl, n-hexyl, methyloxy, ethyloxy, propyloxy, vinyl, allyl, phenyloxy, tolyloxy, benzyloxy, phenethyloxy, phenyl, biphenylyl, terphenylyl, naphthyl, phenanthrenyl, fluorenyl, indenyl, pyrenyl, perylenyl, fluoranthenyl, triphenylenyl, pyridyl, pyrimidinyl, triazinyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, carbolinyl, styryl, naphthylvinyl, acetyl and benzoyl;Ar₁₂ and Ar₁₃ may be the same or different, and represent a hydrogen atom, a substituted or unsubstituted aromatic hydrocarbon group, or a substituted or unsubstituted condensed polycyclic aromatic group; Ar₁₄ represents a substituted or unsubstituted aromatic heterocyclic group;and R₁₀ to R₁₃ may be the same or different, and represent a hydrogen atom, a deuterium atom, a fluorine atom, a chlorine atom, cyano, trifluoromethyl, linear or branched alkyl of 1 to 6 carbon atoms, a substituted or unsubstituted aromatic hydrocarbon group, a substituted or unsubstituted aromatic heterocyclic group, or a substituted or unsubstituted condensed polycyclic aromatic group, where Ar₁₂ and Ar₁₃ are not simultaneously a hydrogen atom.

Specific examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₈ in the general formula (1) and the general formula (1a) include phenyl, biphenylyl, terphenylyl, naphthyl, anthracenyl, phenanthrenyl, fluorenyl, indenyl, pyrenyl, perylenyl, fluoranthenyl, triphenylenyl, pyridyl, pyrimidinyl, triazinyl, furyl, pyrrolyl, thienyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, naphthyridinyl, phenanthrolinyl, acridinyl, and carbolinyl.

Ar₃ and Ar₄ may bind to each other to form a ring via a linking group, such as a single bond, substituted or unsubstituted methylene, an oxygen atom, a sulfur atom, or a monosubstituted amino group. Ar₃ or Ar₄ may bind to the benzene ring binding with -NAr₃Ar₄ group to form a ring via a linking group such as a single bond, substituted or unsubstituted methylene, an oxygen atom, a sulfur atom, or a monosubstituted amino group.

Specific examples of the "substituent" in the "substituted aromatic hydrocarbon group", the "substituted aromatic heterocyclic group", or the "substituted condensed polycyclic aromatic group" represented by Ar₄ to Ar₈ in the general formula (1) and the general formula (1a) include a deuterium atom; cyano; nitro; halogen atoms such as a fluorine atom, a chlorine atom, a bromine atom, and an iodine atom; linear or branched alkyls of 1 to 6 carbon atoms such as methyl, ethyl, n-propyl, isopropyl, n-butyl, isobutyl, tert-butyl, n-pentyl, isopentyl, neopentyl, and n-hexyl; linear or branched alkyloxys of 1 to 6 carbon atoms such as methyloxy, ethyloxy, and propyloxy; alkenyls such as vinyl and allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthrenyl, fluorenyl, indenyl, pyrenyl, perylenyl, fluoranthenyl, and triphenylenyl; aromatic heterocyclic groups such as pyridyl, pyrimidinyl, triazinyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, and carbolinyl; arylvinyls such as styryl and naphthylvinyl; acyls such as acetyl and benzoyl. These substituents may be further substituted with the exemplified substituents above. These substituents may bind to each other to form a ring via a linking group, such as a single bond, substituted or unsubstituted methylene, an oxygen atom, a sulfur atom, or a monosubstituted amino group.

Specific examples of the "aromatic hydrocarbon", the "aromatic heterocyclic ring", or the "condensed polycyclic aromatics" of the "substituted or unsubstituted aromatic hydrocarbon", the "substituted or unsubstituted aromatic heterocyclic ring", or the "substituted or unsubstituted condensed polycyclic aromatics" in the "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by A₁ in the general formula (2) include benzene, biphenyl, terphenyl, tetrakisphenyl, styrene, naphthalene, anthracene, acenaphthalene, fluorene, phenanthrene, indane, pyrene, triphenylene, pyridine, pyrimidine, triazine, pyrrole, furan, thiophene, quinoline, isoquinoline, benzofuran, benzothiophene, indoline, carbazole, carboline, benzoxazole, benzothiazole, quinoxaline, benzimidazole, pyrazole, dibenzofuran, dibenzothiophene, naphthyridine, phenanthroline, and acridine.

The "divalent group of a substituted or unsubstituted aromatic hydrocarbon", the "divalent group of a substituted or unsubstituted aromatic heterocyclic ring", or the "divalent group of substituted or unsubstituted condensed polycyclic aromatics" represented by A₁ in the general formula (2) is a divalent group that results from the removal of two hydrogen atoms from the above "aromatic hydrocarbon", "aromatic heterocyclic ring", or "condensed polycyclic aromatics".

These divalent groups may have a substituent, and examples of the substituent include the same substituents exemplified as the "substituent" in the "substituted aromatic hydrocarbon group", the "substituted aromatic heterocyclic group", or the "substituted condensed polycyclic aromatic group" represented by Ar₁ to Ar₈ in the general formula (1) and the general formula (1a), and possible embodiments may also be the same embodiments as the exemplified embodiments.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₉ to Ar₁₀ in the general formula (2) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₈ in the general formula (1) and the general formula (1a). Ar₉ and Ar₁₀ may bind to each other to form a ring via a linking group, such as a single bond, substituted or unsubstituted methylene, an oxygen atom, a sulfur atom, or a monosubstituted amino group.

These groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by Ar₁ to Ar₈ in the general formula (1) and the general formula (1a), and possible embodiments may also be the same embodiments as the exemplified embodiments.

Specific examples of the "linear or branched alkyl of 1 to 6 carbon atoms", the "cycloalkyl of 5 to 10 carbon atoms", or the "linear or branched alkenyl of 2 to 6 carbon atoms" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₁ to R₇ in the general formula (2) include methyl, ethyl, n-propyl, isopropyl, n-butyl, isobutyl, tert-butyl, n-pentyl, isopentyl, neopentyl, n-hexyl, cyclopentyl, cyclohexyl, 1-adamantyl, 2-adamantyl, vinyl, allyl, isopropenyl, and 2-butenyl. These groups may bind to each other to form a ring via a linking group, such as a single bond, substituted or unsubstituted methylene, an oxygen atom, a sulfur atom, or a monosubstituted amino group. These groups (R₁ to R₇) may bind to the benzene ring to which these groups (R₁ to R₇) directly bind to form a ring via a linking group such as substituted or unsubstituted methylene, an oxygen atom, a sulfur atom, or a monosubstituted amino group.

Specific examples of the "substituent" in the "linear or branched alkyl of 1 to 6 carbon atoms that has a substituent", the "cycloalkyl of 5 to 10 carbon atoms that has a substituent", or the "linear or branched alkenyl of 2 to 6 carbon atoms that has a substituent" represented by R₁ to R₇ in the general formula (2) include a deuterium atom; cyano; nitro; halogen atoms such as a fluorine atom, a chlorine atom, a bromine atom, and an iodine atom; linear or branched alkyloxys of 1 to 6 carbon atoms such as methyloxy, ethyloxy, and propyloxy; alkenyls such as vinyl and allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthrenyl, fluorenyl, indenyl, pyrenyl, perylenyl, fluoranthenyl, and triphenylenyl; aromatic heterocyclic groups such as pyridyl, pyrimidinyl, triazinyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, and carbolinyl; disubstituted amino groups substituted by an aromatic hydrocarbon group or a condensed polycyclic aromatic group, such as diphenylamino and dinaphthylamino; disubstituted amino groups substituted by an aromatic heterocyclic group, such as dipyridylamino and dithienylamino; and disubstituted amino groups substituted by substituents selected from aromatic hydrocarbon groups, condensed polycyclic aromatic groups, and aromatic heterocyclic groups. These substituents may be further substituted with the exemplified substituents above. These substituents may bind to each other to form a ring via a linking group such as a single bond, substituted or unsubstituted methylene, an oxygen atom, a sulfur atom, or a monosubstituted amino group.

Specific examples of the "linear or branched alkyloxy of 1 to 6 carbon atoms" or the "cycloalkyloxy of 5 to 10 carbon atoms" in the "linear or branched alkyloxy of 1 to 6 carbon atoms that may have a substituent" or the "cycloalkyloxy of 5 to 10 carbon atoms that may have a substituent" represented by R₁ to R₇ in the general formula (2) include methyloxy, ethyloxy, n-propyloxy, isopropyloxy, n-butyloxy, tert-butyloxy, n-pentyloxy, n-hexyloxy, cyclopentyloxy, cyclohexyloxy, cycloheptyloxy, cyclooctyloxy, 1-adamantyloxy, and 2-adamantyloxy. These groups may bind to each other to form a ring via a linking group such as a single bond, substituted or unsubstituted methylene, an oxygen atom, a sulfur atom, or a monosubstituted amino group. These groups (R₁ to R₇) may bind to the benzene rings to which these groups (R₁ to R₇) directly bind to form a ring via a linking group such as substituted or unsubstituted methylene, an oxygen atom, a sulfur atom, or a monosubstituted amino group.

These groups may have a substituent. Examples of the substituent include the same groups exemplified as the "substituent" in the "linear or branched alkyl of 1 to 6 carbon atoms that has a substituent", the "cycloalkyl of 5 to 10 carbon atoms that has a substituent", or the "linear or branched alkenyl of 2 to 6 carbon atoms that has a substituent" represented by R₁ to R₇ in the general formula (2), and possible embodiments may also be the same embodiments as the exemplified embodiments.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R₁ and R₇ in the general formula (2) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₈ in the general formula (1) and the general formula (1a). These groups may bind to each other to form a ring via a linking group, such as a single bond, substituted or unsubstituted methylene, an oxygen atom, a sulfur atom, or a monosubstituted amino group. These groups (R₁ to R₇) may bind to the benzene rings to which these groups (R₁ to R₇) directly bind to form a ring via a linking group such as substituted or unsubstituted methylene, an oxygen atom, a sulfur atom, or a monosubstituted amino group.

Specific examples of the "substituent" in the "substituted aromatic hydrocarbon group", the "substituted aromatic heterocyclic group", and the "substituted condensed polycyclic aromatic group" represented by R₁ to R₇ in the general formula (2) include a deuterium atom; cyano; nitro; halogen atoms such as a fluorine atom, a chlorine atom, a bromine atom, and an iodine atom; linear or branched alkyls of 1 to 6 carbon atoms such as methyl, ethyl, n-propyl, isopropyl, n-butyl, isobutyl, tert-butyl, n-pentyl, isopentyl, neopentyl, and n-hexyl; linear or branched alkyloxys of 1 to 6 carbon atoms such as methyloxy, ethyloxy, and propyloxy; alkenyls such as vinyl and allyl; aryloxys such as phenyloxy and tolyloxy; arylalkyloxys such as benzyloxy and phenethyloxy; aromatic hydrocarbon groups or condensed polycyclic aromatic groups such as phenyl, biphenylyl, terphenylyl, naphthyl, anthracenyl, phenanthrenyl, fluorenyl, indenyl, pyrenyl, perylenyl, fluoranthenyl, and triphenylenyl; aromatic heterocyclic groups such as pyridyl, pyrimidinyl, triazinyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, and carbolinyl; arylvinyls such as styryl and naphthylvinyl; acyls such as acetyl and benzoyl; silyls such as trimethylsilyl and triphenylsilyl; disubstituted amino groups substituted by an aromatic hydrocarbon group or a condensed polycyclic aromatic group, such as diphenylamino and dinaphthylamino; disubstituted amino groups substituted by an aromatic heterocyclic group, such as dipyridylamino and dithienylamino; and disubstituted amino groups substituted by substituents selected from aromatic hydrocarbon groups, condensed polycyclic aromatic groups, and aromatic heterocyclic groups. These substituents may be further substituted with the exemplified substituents above. These substituents may bind to each other to form a ring via a linking group such as a single bond, substituted or unsubstituted methylene, an oxygen atom, a sulfur atom, or a monosubstituted amino group.

Specific examples of the "aryloxy group" in the "substituted or unsubstituted aryloxy group" represented by R₁ to R₇ in the general formula (2) include phenyloxy, biphenylyloxy, terphenylyloxy, naphthyloxy, anthracenyloxy, phenanthrenyloxy, fluorenyloxy, indenyloxy, pyrenyloxy, and perylenyloxy. These substituents may bind to each other to form a ring via a linking group such as a single bond, substituted or unsubstituted methylene, an oxygen atom, a sulfur atom, or a monosubstituted amino group. These groups (R₁ to R₇) may bind to the benzene rings to which these groups (R₁ to R₇) directly bind to form a ring via a linking group such as substituted or unsubstituted methylene, an oxygen atom, a sulfur atom, or a monosubstituted amino group.

These groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by R₁ to R₇ in the general formula (2), and possible embodiments may also be the same embodiments as the exemplified embodiments.

Examples of the "aromatic hydrocarbon group", the "aromatic heterocyclic group", or the "condensed polycyclic aromatic group" in the "disubstituted amino group substituted by substituents selected from aromatic hydrocarbon group, aromatic heterocyclic group, and condensed polycyclic aromatic group" represented by R₁ to R₄ in the general formula (2) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₈ in the general formula (1) and the general formula (1a).

These groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by R₁ to R₇ in the general formula (2), and possible embodiments may also be the same embodiments as the exemplified embodiments.

As for the "disubstituted amino group substituted by substituents selected from aromatic hydrocarbon group, aromatic heterocyclic group, and condensed polycyclic aromatic group" represented by R₁ to R₄ in the general formula (2), these groups (R₁ to R₄) may bind to each other to form a ring, via a single bond, substituted or unsubstituted methylene, an oxygen atom, a sulfur atom, or a monosubstituted amino group, and via the "aromatic hydrocarbon group", the "aromatic heterocyclic group", or the "condensed polycyclic aromatic group" of these groups (R₁ to R₄). These groups (R₁ to R₄) may bind to the benzene ring to which these groups (R₁ to R₄) directly bind to form a ring, via a linking group, such as substituted or unsubstituted methylene, an oxygen atom, a sulfur atom, or a monosubstituted amino group, and via the "aromatic hydrocarbon group", the "aromatic heterocyclic group", or the "condensed polycyclic aromatic group" of these groups (R₁ to R₄).

Examples of the "linear or branched alkyl of 1 to 6 carbon atoms", the "cycloalkyl of 5 to 10 carbon atoms", or the "linear or branched alkenyl of 2 to 6 carbon atoms" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₈ and R₉ in the general formula (2) include the same groups exemplified as the groups for the "linear or branched alkyl of 1 to 6 carbon atoms", the "cycloalkyl of 5 to 10 carbon atoms", or the "linear or branched alkenyl of 2 to 6 carbon atoms" in the "linear or branched alkyl of 1 to 6 carbon atoms that may have a substituent", the "cycloalkyl of 5 to 10 carbon atoms that may have a substituent", or the "linear or branched alkenyl of 2 to 6 carbon atoms that may have a substituent" represented by R₁ to R₇ in the general formula (2). These groups may bind to each other to form a ring via a linking group, such as a single bond, substituted or unsubstituted methylene, an oxygen atom, a sulfur atom, or a monosubstituted amino group.

These groups may have a substituent. Examples of the substituent include the same groups exemplified as the "substituent" in the "linear or branched alkyl of 1 to 6 carbon atoms that has a substituent", the "cycloalkyl of 5 to 10 carbon atoms that has a substituent", the "substituted aromatic hydrocarbon group", the "substituted aromatic heterocyclic group", or the "substituted condensed polycyclic aromatic group" represented by R₁ to R₇ in the general formula (2), and possible embodiments may also be the same embodiments as the exemplified embodiments.

Examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R₈ and R₉ in the general formula (2) include the same groups exemplified as the groups for the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by Ar₁ to Ar₈ in the general formula (1) and the general formula (1a). These groups may bind to each other to form a ring via a linking group, such as a single bond, substituted or unsubstituted methylene, an oxygen atom, a sulfur atom, or a monosubstituted amino group.

These groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by R₁ to R₇ in the general formula (2), and possible embodiments may also be the same embodiments as the exemplified embodiments.

Examples of the "aryloxy" in the "substituted or unsubstituted aryloxy" represented by R₈ and R₉ in the general formula (2) include the same groups exemplified as the groups for the "aryloxy" in the "substituted or unsubstituted aryloxy" represented by R₁ to R₇ in the general formula (2), and possible embodiments may also be the same embodiments as the exemplified embodiments. These groups may bind to each other to form a ring via a single bond, substituted or unsubstituted methylene, an oxygen atom, or a sulfur atom, or a monosubstituted amino group.

These groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by R₁ to R₇ in the general formula (2), and possible embodiments may also be the same embodiments as the exemplified embodiments.

Examples of the "substituent" in the "monosubstituted amino group" as the linking group in the general formula (2) include the same groups exemplified as the "linear or branched alkyl of 1 to 6 carbon atoms", the "cycloalkyl of 5 to 10 carbon atoms", the "aromatic hydrocarbon group", the "aromatic heterocyclic group", or the "condensed polycyclic aromatic group" in the "linear or branched alkyl of 1 to 6 carbon atoms that may have a substituent", the "cycloalkyl of 5 to 10 carbon atoms that may have a substituent", the "substituted or unsubstituted aromatic hydrocarbon group", the "substituted or unsubstituted aromatic heterocyclic group", or the "substituted or unsubstituted condensed polycyclic aromatic group" represented by R₁ to R₇ in the general formula (2).

These groups may have a substituent. Examples of the substituent include the same substituents exemplified as the "substituent" in the "linear or branched alkyl of 1 to 6 carbon atoms that has a substituent", the "cycloalkyl of 5 to 10 carbon atoms that has a substituent", the "substituted aromatic hydrocarbon group", the "substituted aromatic heterocyclic group", or the "substituted condensed polycyclic aromatic group" represented by R₁ to R₇ in the general formula (2), and possible embodiments may also be the same embodiments as the exemplified embodiments.

Specific examples of the "aromatic hydrocarbon group" or the "condensed polycyclic aromatic group" in the "substituted or unsubstituted aromatic hydrocarbon group" or the "substituted or unsubstituted condensed polycyclic aromatic group" represented by Ar₁₁, Ar₁₂ and Ar₁₃ in the general formula (3) include phenyl, biphenylyl, terphenylyl, quaterphenyl, styryl, naphthyl, anthracenyl, acenaphthenyl, phenanthrenyl, fluorenyl, indenyl, pyrenyl, perylenyl, fluoranthenyl and triphenylenyl.

Further, these groups may have a substituent. Examples of the substituent of Ar₁₂ and Ar₁₃ include the same groups exemplified as the "substituent" in the "substituted aromatic hydrocarbon group", the "substituted aromatic heterocyclic group", or the "substituted condensed polycyclic aromatic group" represented by Ar₁ to Ar₈ in the general formula (1) and the general formula (1a), and possible embodiments may also be the same embodiments as the exemplified embodiments.

The substituents in the substituted aromatic hydrocarbon group, or the substituted condensed polycyclic aromatic group of Ar₁₁ are selected from the group consisting of a deuterium atom, cyano, nitro, a fluorine atom, a chlorine atom, a bromine atom, an iodine atom, methyl, ethyl, n-propyl, isopropyl, n-butyl, isobutyl, tert-butyl, n-pentyl, isopentyl, neopentyl, n-hexyl, methyloxy, ethyloxy, propyloxy, vinyl, allyl, phenyloxy, tolyloxy, benzyloxy, phenethyloxy, phenyl, biphenylyl, terphenylyl, naphthyl, phenanthrenyl, fluorenyl, indenyl, pyrenyl, perylenyl, fluoranthenyl, triphenylenyl, pyridyl, pyrimidinyl, triazinyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, carbolinyl, styryl, naphthylvinyl, acetyl and benzoyl.

Specific examples of the "aromatic heterocyclic group" in the "substituted or unsubstituted aromatic heterocyclic group" represented by Ar₁₄ in the general formula (3) include triazinyl, pyridyl, pyrimidinyl, furyl, pyrrolyl, thienyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, naphthyridinyl, phenanthrolinyl, acridinyl, and carbolinyl.

Further, these groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by Ar₁ to Ar₈ in the general formula (1) and the general formula (1a), and possible embodiments may also be the same embodiments as the exemplified embodiments.

Specific examples of the "linear or branched alkyl of 1 to 6 carbon atoms" represented by R₁₀ to R₁₃ in the general formula (3) include methyl, ethyl, n-propyl, isopropyl, n-butyl, 2-methylpropyl, tert-butyl, n-pentyl, 3-methylbutyl, tert-pentyl, n-hexyl, isohexyl and tert-hexyl.

Specific examples of the "aromatic hydrocarbon group", the "aromatic heterocyclic group", or the "condensed polycyclic aromatic group" in the "substituted or unsubstituted aromatic hydrocarbon group", the "substituted or unsubstituted aromatic heterocyclic group", or the "substituted or unsubstituted condensed polycyclic aromatic group" represented by R₁₀ to R₁₃ in the general formula (3) include phenyl, biphenylyl, terphenylyl, quaterphenyl, styryl, naphthyl, anthracenyl, acenaphthenyl phenanthrenyl, fluorenyl, indenyl, pyrenyl, perylenyl, fluoranthenyl, triphenylenyl, triazinyl, pyridyl, pyrimidinyl, furyl, pyrrolyl, thienyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, naphthyridinyl, phenanthrolinyl, acridinyl, and carbolinyl.

Further, these groups may have a substituent. Examples of the substituent include the same groups exemplified as the "substituent" in the "substituted aromatic hydrocarbon group", the "substituted aromatic heterocyclic group", or the "substituted condensed polycyclic aromatic group" represented by Ar₁ to Ar₈ in the general formula (1) and the general formula (1a), and possible embodiments may also be the same embodiments as the exemplified embodiments.

The "substituent" in the "substituted aromatic hydrocarbon group", the "substituted aromatic heterocyclic group", or the "substituted condensed polycyclic aromatic group" represented by Ar₁ to Ar₈ in the general formula (1) and the general formula (1a) is preferably a deuterium atom, the "linear or branched alkyl of 1 to 6 carbon atoms that may have a substituent", the "linear or branched alkenyl of 2 to 6 carbon atoms that may have a substituent", the "substituted or unsubstituted aromatic hydrocarbon group", or the "substituted or unsubstituted condensed polycyclic aromatic group", far preferably, a deuterium atom, phenyl, biphenylyl, naphthyl, or vinyl. It is also preferable that these groups bind to each other via a single bond to form a condensed aromatic ring.

In the general formula (1) and the general formula (1a), n1 represents 0 or 1 to 2, in which the case where n1 is 0 shows that the two diarylamino benzene rings are bonded directly (via a single bond), the case where n1 is 1 shows that the two diarylamino benzene rings are bonded via one phenylene group, and the case where n1 is 2 shows that the two diarylamino benzene rings are bonded via two phenylene groups (a biphenylene group).

In the general formula (1) and the general formula (1a), it is preferable that n1 is 0, that is, the two diarylamino benzene rings are bonded directly (via a single bond).

In the general formula (1) and the general formula (1a), it is also preferable that Ar₃ or Ar₄ may bind to the benzene ring to which -NAr₃Ar₄ group (a diarylamino group comprising Ar₃, Ar₄, and a nitrogen atom to which Ar₃ and Ar₄ bind) bind, via a linking group such as a single bond, substituted or unsubstituted methylene, an oxygen atom, a sulfur atom, or a monosubstituted amino group to form a ring. In this case, the bonding position in the benzene ring is preferably adjacent to -NAr₃Ar₄ group.

A₁ in the general formula (2) is preferably the "divalent group of a substituted or unsubstituted aromatic hydrocarbon" or a single bond, far preferably, a divalent group that results from the removal of two hydrogen atoms from benzene, biphenyl, or naphthalene; or a single bond, particularly preferably a single bond.

Ar₉ and Ar₁₀ in the general formula (2) are preferably phenyl, biphenylyl, naphthyl, fluorenyl, indenyl, pyridyl, dibenzofuranyl, pyridobenzofuranyl.

Ar₉ and Ar₁₀ in the general formula (2) may bind to each other to form a ring via a single bond, substituted or unsubstituted methylene, an oxygen atom, a sulfur atom, or a monosubstituted amino group and via the substituent of these groups or directly.

It is preferable that at least one of R₁ to R₄ in the general formula (2) is a "disubstituted amino group substituted with a group selected from an aromatic hydrocarbon group, an aromatic heterocyclic group, or a condensed polycyclic aromatic group", and the "aromatic hydrocarbon group", the "aromatic heterocyclic group", or the "condensed polycyclic aromatic group" in this case is preferably phenyl, biphenylyl, naphthyl, fluorenyl, indenyl, pyridyl, dibenzofuranyl, or pyridobenzofuranyl.

In the general formula (2), an embodiment where adjacent two or all of R₁ to R₄ are vinyls, and the adjacent two vinyls bind to each other via a single bond to form a condensed ring, that is an embodiment where the groups form a naphthalene ring or a phenanthrene ring with the benzene ring to which R₁ to R₄ bind, is also preferable.

In the general formula (2), an embodiment where any one of R₁ to R₄ is the "aromatic hydrocarbon group", and binds to the benzene ring to which R₁ to R₄ bind to form a ring, via a linking group such as substituted or unsubstituted methylene, an oxygen atom, a sulfur atom, or a monosubstituted amino group is preferable. In this case, an embodiment where the "aromatic hydrocarbon group" is phenyl, and binds to the benzene ring to which R₁ to R₄ bind to form a ring, via a linking group such as an oxygen atom, a sulfur atom, or a monosubstituted amino group, that is, an embodiment where the groups form a dibenzofuran ring or a dibenzothiophene ring with the benzene ring to which R₁ to R₄ bind, is particularly preferable.

In the general formula (2), an embodiment where any one of R₅ to R₇ is the "aromatic hydrocarbon group", and binds to the benzene ring to which R₅ to R₇ bind to form a ring, via a linking group such as substituted or unsubstituted methylene, an oxygen atom, a sulfur atom, or a monosubstituted amino group is preferable. In this case, an embodiment where the "aromatic hydrocarbon group" is phenyl, and binds to the benzene ring to which R₅ to R₇ bind to form a ring, via a linking group such as an oxygen atom, a sulfur atom, or a monosubstituted amino group, that is, an embodiment where the groups form a dibenzofuran ring or a dibenzothiophene ring is particularly preferable.

In the amine derivative having a condensed ring structure of the general formula (2), as the embodiment where R₁ to R₇ bind to each other to form a ring, or the embodiment where R₁ to R₇ bind to the benzene rings to which R₁ to R₇ bind, to form a ring, as described above, embodiments of the following general formulae (2a-a), (2a-b), (2b-a), (2b-b), (2b-c), (2b-d), (2c-a), and (2c-b) are preferably used.

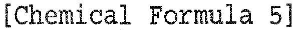

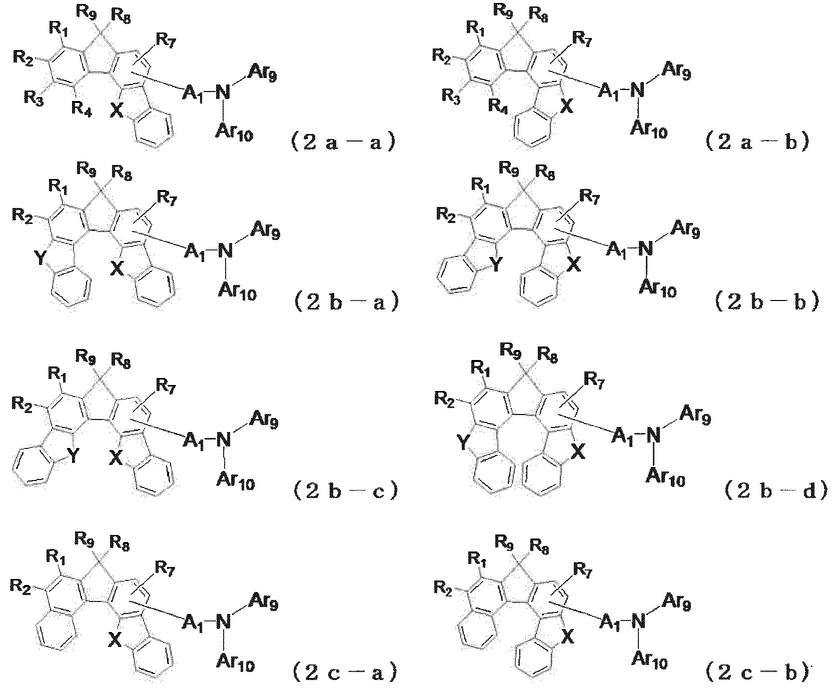

In the formulae, X and Y may be the same or different and represent an oxygen atom or a sulfur atom, and A₁, Ar₉, Ar₁₀, R₁ to R₄, R₇, R₈ and R₉ have the same meanings as shown for the general formula (2).

R₈ and R₉ in the general formula (2) are preferably the "substituted or unsubstituted aromatic hydrocarbon group", the "substituted or unsubstituted an oxygen-containing aromatic heterocyclic group", or the "substituted or unsubstituted condensed polycyclic aromatic group", further preferably phenyl, naphthyl, phenanthrenyl, pyridyl, quinolyl, isoquinolyl, or dibenzofuranyl, and particularly preferably phenyl.

An embodiment where R₈ and R₉ bind to each other via a linking group such as a single bond, substituted or unsubstituted methylene, an oxygen atom, a sulfur atom, or a monosubstituted amino group to form a ring is preferable, and an embodiment where the groups bind to each other via a single bond to form a ring is particularly preferable.

In the amine derivative having a condensed ring structure of the general formula (2), as the embodiment where R₈ and R₉ bind to each other to form a ring as described above, embodiments of the following general formulae (2a-a1), (2a-b1), (2b-a1), (2b-b1), (2b-c1), (2b-d1), (2c-a1), and (2c-b1) are preferably used.

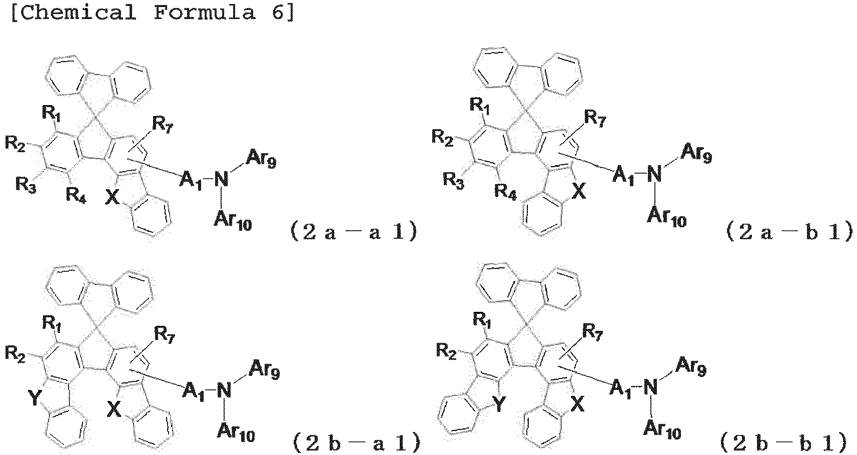

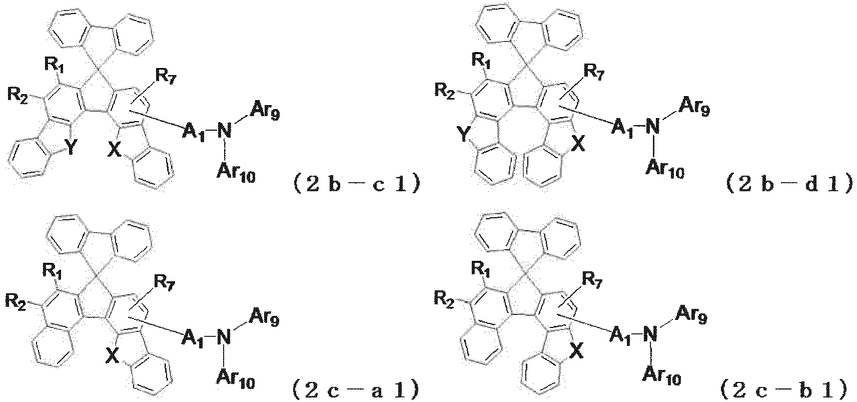

In the formulae, X and Y may be the same or different and represent an oxygen atom or a sulfur atom, and A₁, Ar₉, Ar₁₀, R₁ to R₄, and R₇ have the same meanings as shown for the general formula (2).

Ar₁₁ in the general formula (3) is preferably phenyl, biphenylyl, naphthyl, anthracenyl, acenaphthenyl, phenanthrenyl, fluorenyl, indenyl, pyrenyl, perylenyl, fluoranthenyl or triphenylenyl, and further preferably phenyl, biphenylyl, naphthyl, anthracenyl, phenanthrenyl, pyrenyl, fluoranthenyl or triphenylenyl. The phenyl group preferably has a substituent according to the claims.

Ar₁₂ in the general formula (3) is preferably phenyl that has a substituent. The substituent of the phenyl in this case is preferably an aromatic hydrocarbon group, such as phenyl, biphenylyl, and terphenyl, or a condensed polycyclic aromatic group, such as naphthyl, anthracenyl, acenaphthenyl, phenanthrenyl, fluorenyl, indenyl, pyrenyl, perylenyl, fluoranthenyl, and triphenylenyl, and further preferably phenyl, naphthyl, anthracenyl, phenanthrenyl, pyrenyl, fluoranthenyl, or triphenylenyl.

Ar₁₃ in the general formula (3) is preferably phenyl that has a substituent. The substituent of the phenyl in this case is preferably an aromatic hydrocarbon group, such as phenyl, biphenylyl, and terphenyl, or a condensed polycyclic aromatic group, such as naphthyl, anthracenyl, acenaphthenyl, phenanthrenyl, fluorenyl, indenyl, pyrenyl, perylenyl, fluoranthenyl, and triphenylenyl, and further preferably phenyl, naphthyl, anthracenyl, phenanthrenyl, pyrenyl, fluoranthenyl, or triphenylenyl.

In the general formula (3), it is preferable that Ar₁₁ and Ar₁₂ are not the same as each other from the viewpoint of thin film stability. When Ar₁₁ and Ar₁₂ are not the same groups, the groups may have different substituents and may be substituted on different positions.

In the general formula (3), Ar₁₂ and Ar₁₃ may be the same groups, but there may be a possibility that the compound is easily crystallized due to the high symmetry of the entire molecule, and from the viewpoint of thin film stability, it is preferable that Ar₁₂ and Ar₁₃ are not the same as each other, and Ar₁₂ and Ar₁₃ are not simultaneously a hydrogen atom.

In the general formula (3), it is preferable that one of Ar₁₂ and Ar₁₃ is a hydrogen atom.

Ar₁₄ in the general formula (3) is preferably a nitrogen-containing heterocyclic group such as triazinyl, pyridyl, pyrimidinyl, pyrrolyl, quinolyl, isoquinolyl, indolyl, carbazolyl, benzoxazolyl, benzothiazolyl, quinoxalinyl, benzoimidazolyl, pyrazolyl, naphthyridinyl, phenanthrolinyl, acridinyl, or carbolinyl, more preferably triazinyl, pyridyl, pyrimidinyl, quinolyl, isoquinolyl, indolyl, quinoxalinyl, benzoimidazolyl, naphthyridinyl, phenanthrolinyl, or acridinyl, particularly preferably pyridyl, pyrimidinyl, quinolyl, isoquinolyl, indolyl, quinoxalinyl, benzoimidazolyl, phenanthrolinyl, or acridinyl.

In the general formula (3), a bonding position of Ar₁₄ in the benzene ring is preferably a meta position with respect to a bonding position of the pyrimidine ring from the viewpoint of stability as a thin film.

Examples of the compound having a pyrimidine ring structure represented by the general formula (3) include compounds having a pyrimidine ring structure of the following general formula (3a) and the general formula (3b) in which a bonding pattern of the substituents is different.

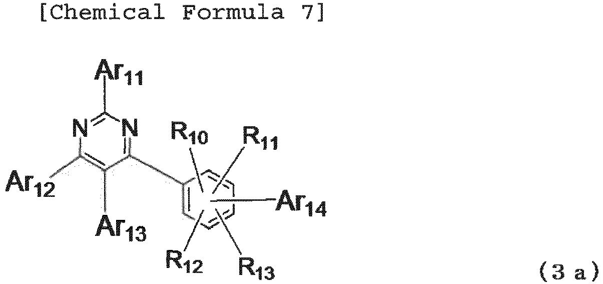

In the formula, Ar₁₁, Ar₁₂, Ar₁₃, Ar₁₄ and R₁₀ to R₁₃ represent the same meanings as described in the above general formula (3).

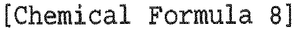

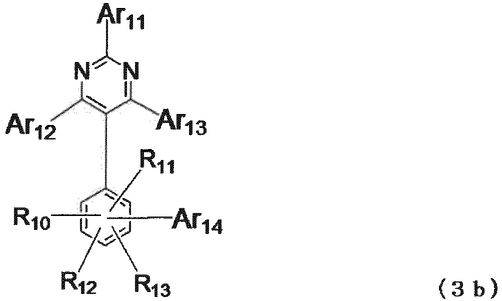

In the formula, Ar₁₁, Ar₁₂, Ar₁₃, Ar₁₄ and R₁₀ to R₁₃ represent the same meanings as described in the above general formula (3).

The arylamine compounds of the general formula (1) and the general formula (1a), for preferred use in the organic EL device of the present invention, can be used as a constitutive material of a hole injection layer, an electron blocking layer, or a hole transport layer of an organic EL device. The arylamine compounds of the general formula (1) and the general formula (1a) have high hole mobility and are therefore preferred compounds as a material of a hole injection layer or a hole transport layer. Further, the arylamine compounds of the general formula (1) and the general formula (1a) have high electron blocking performance, and are therefore preferred compounds as a material of an electron blocking layer.

The amine derivatives of the general formula (2) having a condensed ring structure preferably used in the organic EL device of the present invention can be used as a constitutive material of a light emitting layer of an organic EL device. The compound is excellent in light emission efficiency as compared to the conventional materials, and is a preferred compound as a dopant material for a light emitting layer.

The compounds of the general formula (3) having a pyrimidine ring structure, for preferable use in the organic EL device of the present invention, can be used as a constitutive material of an electron transport layer of an organic EL device.

The compounds of the general formula (3) having a pyrimidine ring structure, excel in electron injection and transport abilities and further excel in stability as a thin film and durability, and are therefore preferred compounds as a material of an electron transport layer.

In the organic EL device of the present invention, materials for an organic EL device having excellent hole and electron injection/transport performances, stability as a thin film, and durability are combined while taking carrier balance that matches the characteristics of a material of a light emitting layer having a specific structure into consideration. Therefore, compared with the conventional organic EL devices, hole transport efficiency to a light emitting layer from a hole transport layer is improved, and electron transport efficiency to a light emitting layer from an electron transport layer is also improved. As a result, luminous efficiency is improved, and also driving voltage is decreased, and thus, durability of the organic EL device can be improved.

Thus, an organic EL device having high luminous efficiency and a long lifetime can be attained. Advantageous Effects of Invention

The organic EL device of the present invention can achieve an organic EL device which can efficiently inject/transport holes into a light emitting layer, and therefore has high efficiency, low driving voltage, and a long lifetime by selecting an arylamine compound having a specific structure, which has excellent hole and electron injection/transport performances, stability as a thin film, and durability, and can effectively exhibit hole injection/transport roles. Further, an organic EL device having high efficiency, low driving voltage, and particularly a long lifetime can be achieved by selecting an arylamine compound having a specific structure, and by combining this compound with a specific electron transport material so as to achieve good carrier balance that matches characteristics of a material of the light emitting layer having a specific structure.

According to the present invention, the luminous efficiency and durability of the conventional organic EL devices can be improved.

### Brief Description of Drawings

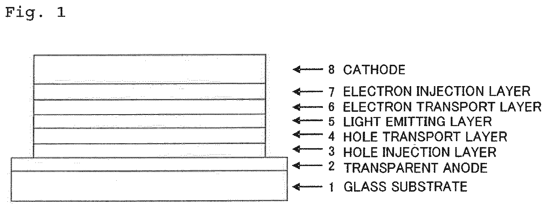

Fig. 1 is a diagram illustrating the configuration of the organic EL devices of Examples 14 to 15 and Comparative Examples 1 to 2.

### Description of Embodiments

The following presents specific examples of preferred compounds among the arylamine compounds of the general formula (1) preferably used in the organic EL device of the present invention. The present invention, however, is not restricted to these compounds.

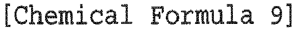

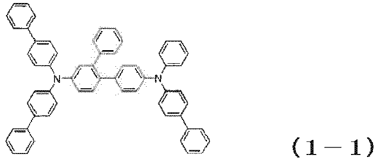

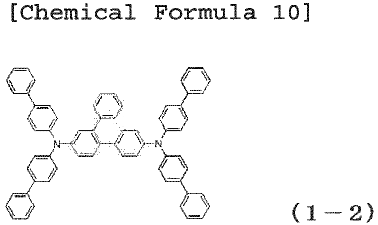

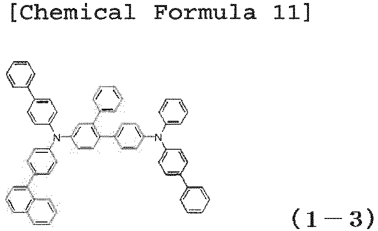

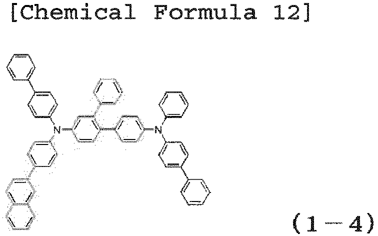

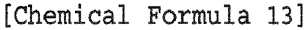

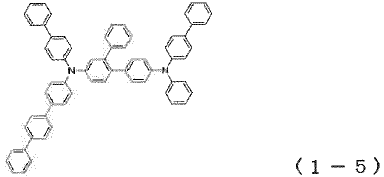

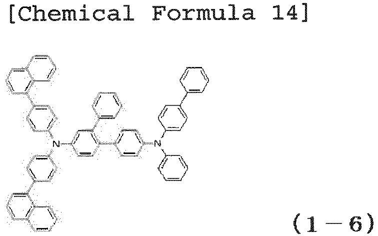

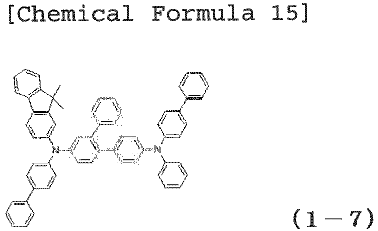

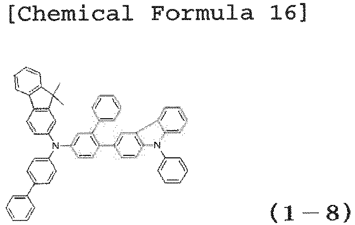

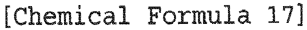

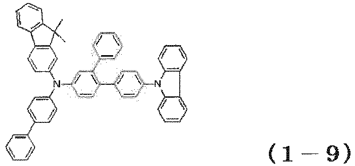

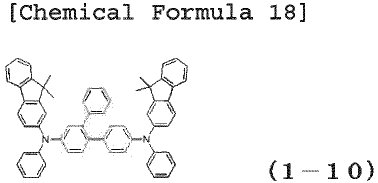

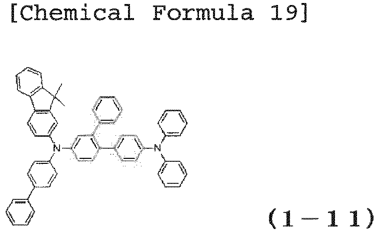

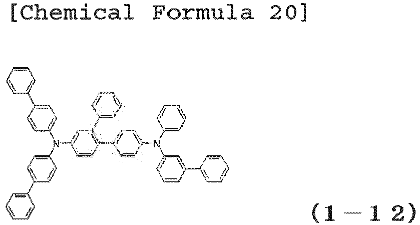

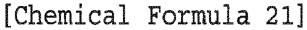

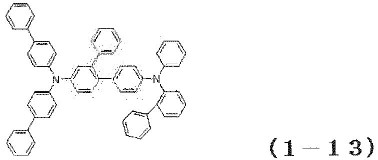

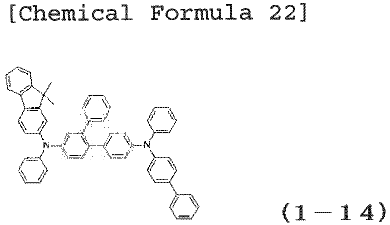

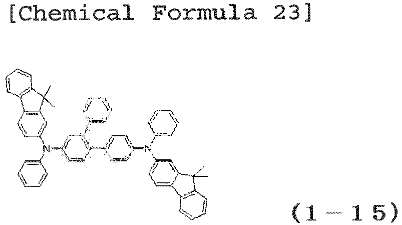

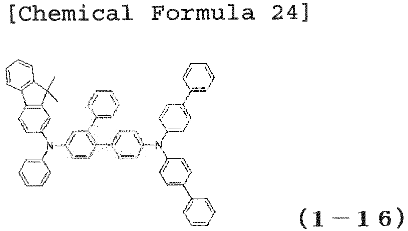

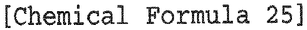

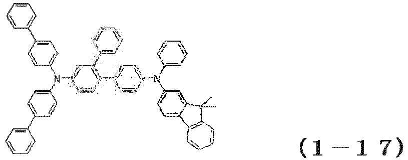

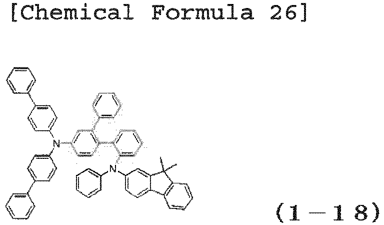

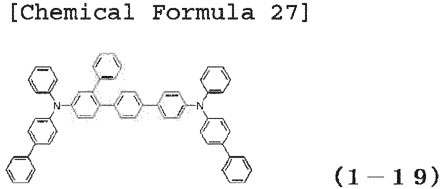

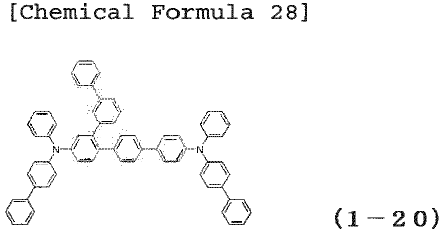

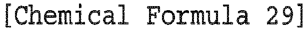

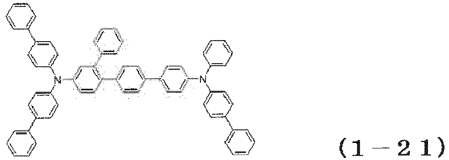

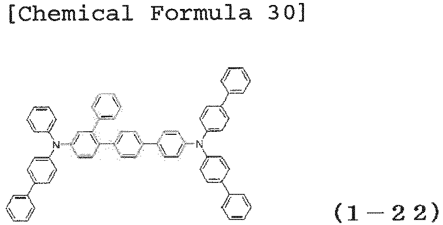

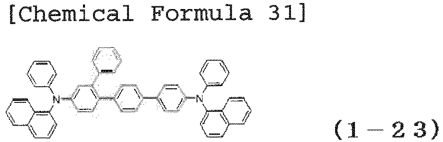

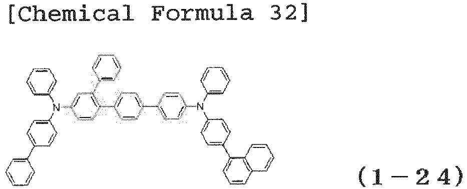

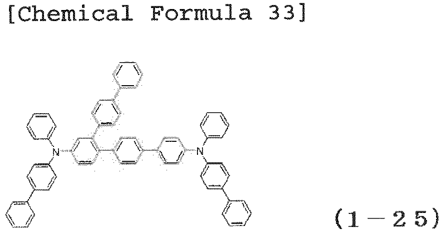

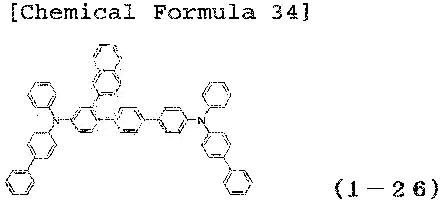

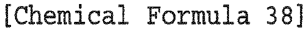

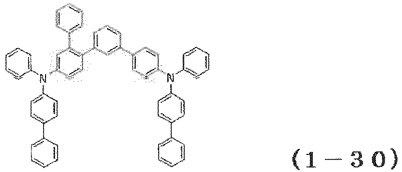

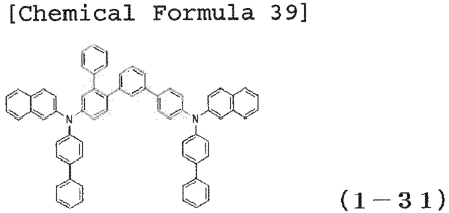

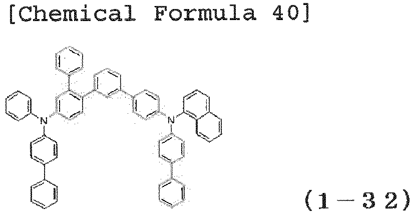

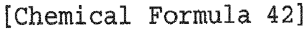

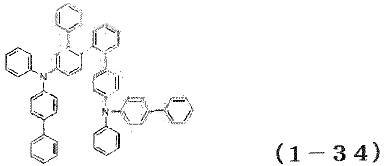

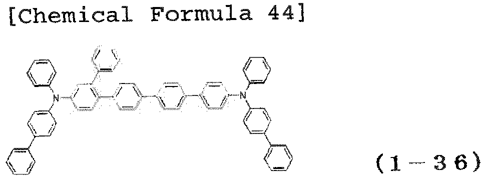

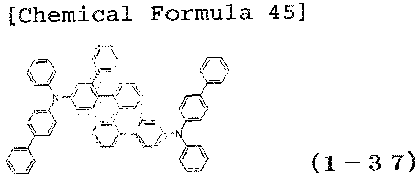

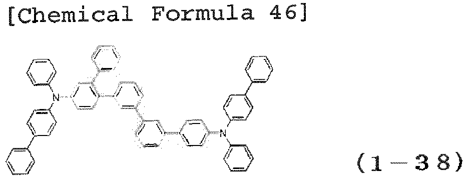

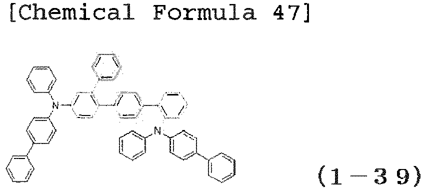

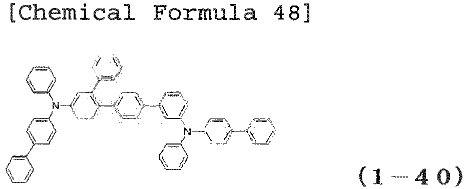

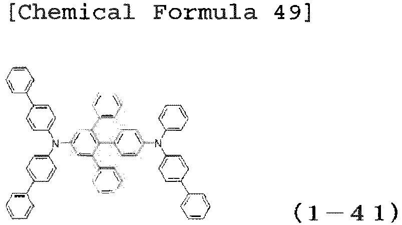

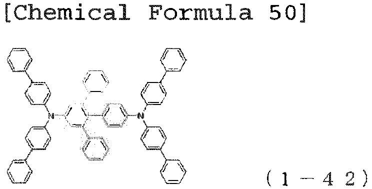

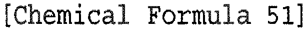

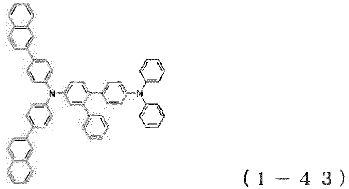

The following presents specific examples of preferred compounds among the amine derivatives of the general formula (2) preferably used in the organic EL device of the present invention and having a condensed ring structure. The present invention, however, is not restricted to these compounds.

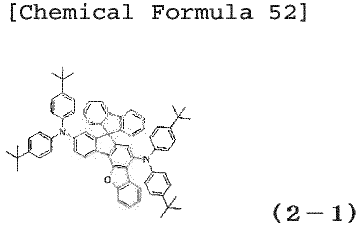

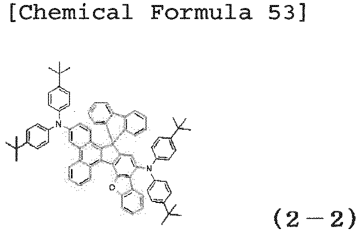

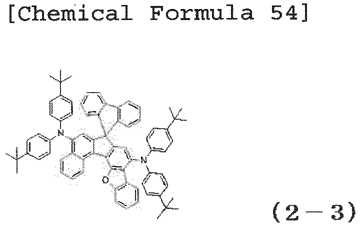

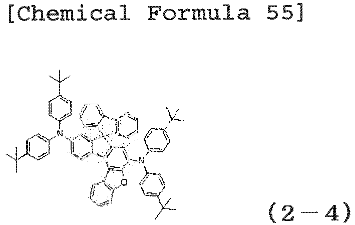

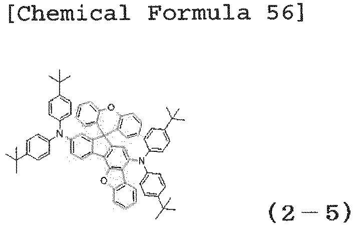

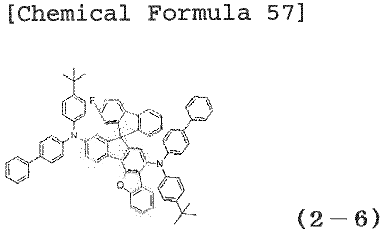

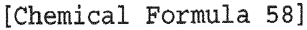

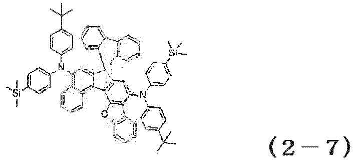

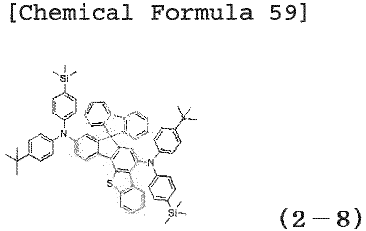

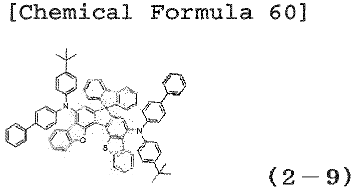

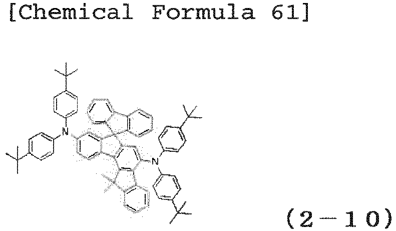

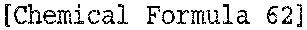

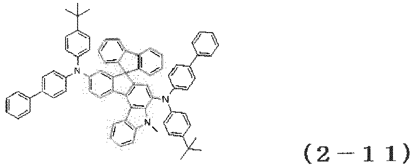

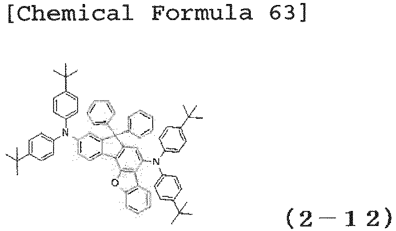

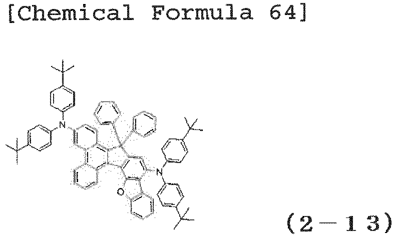

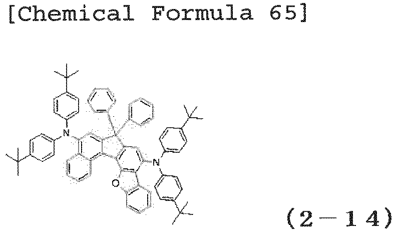

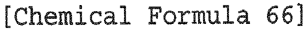

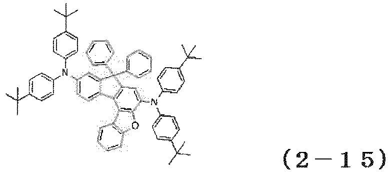

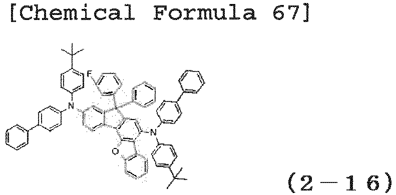

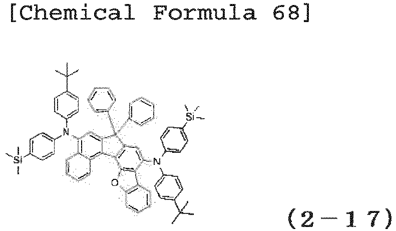

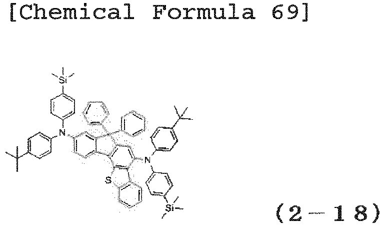

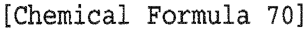

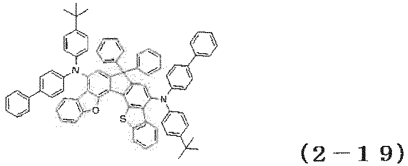

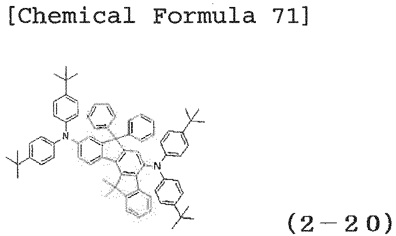

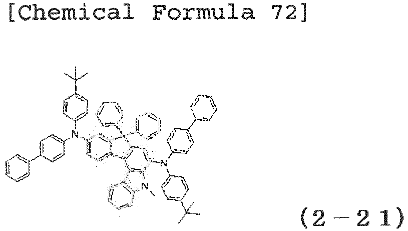

The amine derivatives having a condensed ring structure described above can be synthesized by a known method (refer to PTL 6, for example).

The following presents specific examples of preferred compounds among the compounds of the general formula (3) preferably used in the organic EL device of the present invention and having a pyrimidine ring structure. The present invention, however, is not restricted to these compounds.

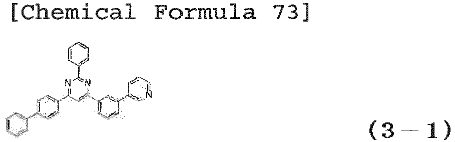

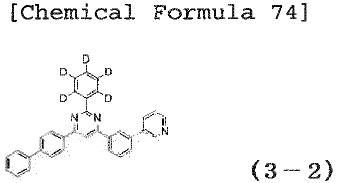

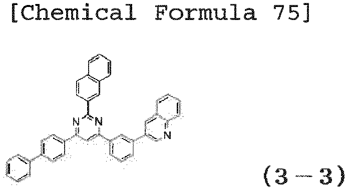

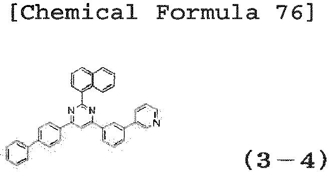

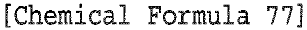

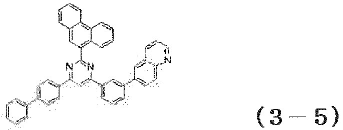

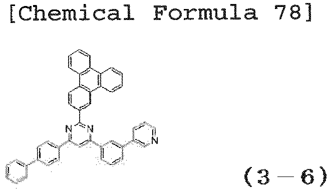

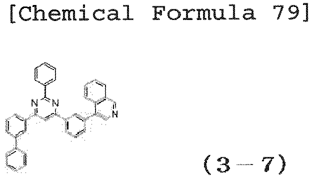

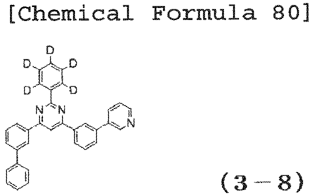

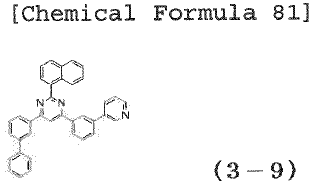

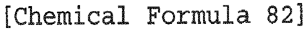

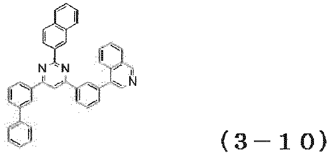

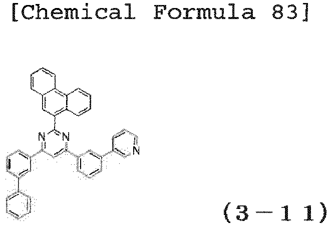

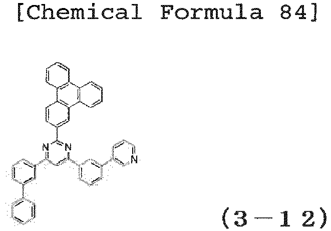

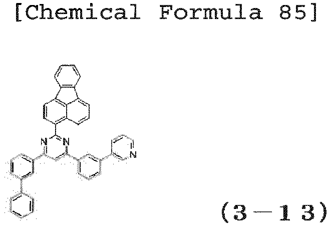

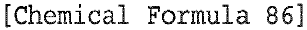

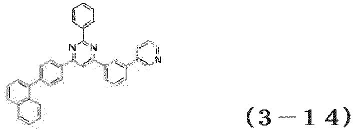

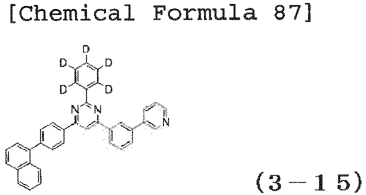

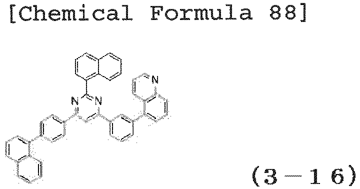

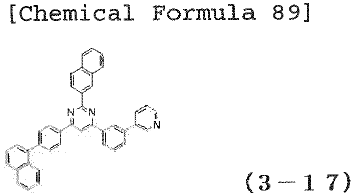

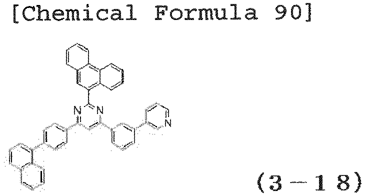

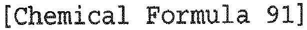

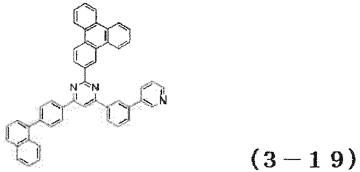

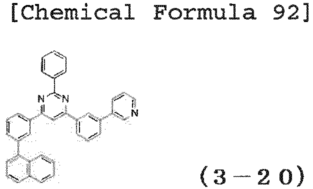

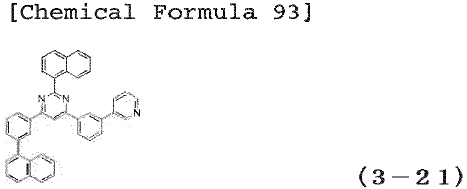

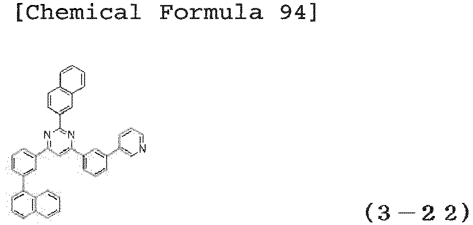

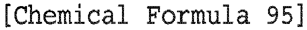

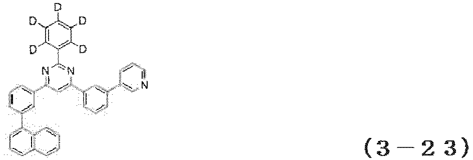

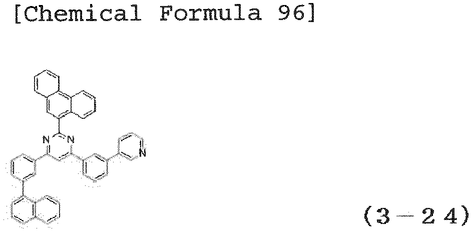

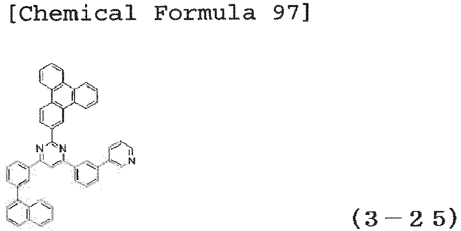

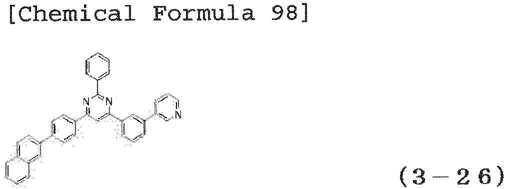

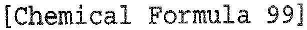

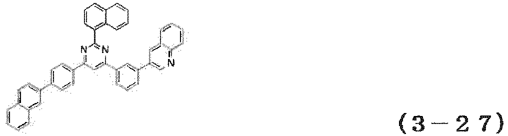

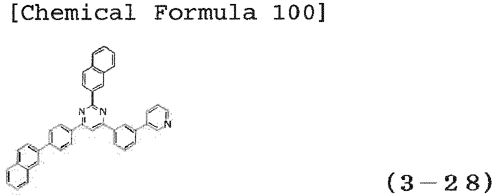

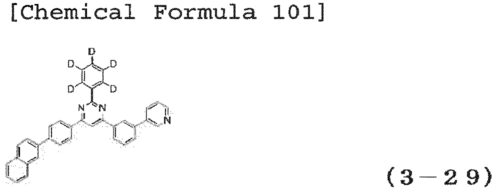

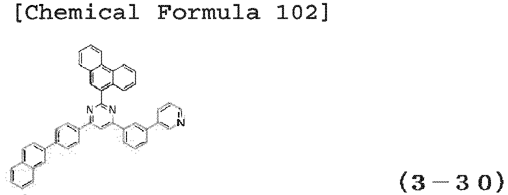

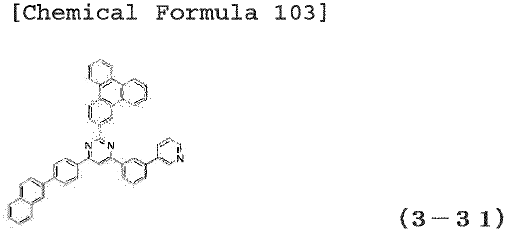

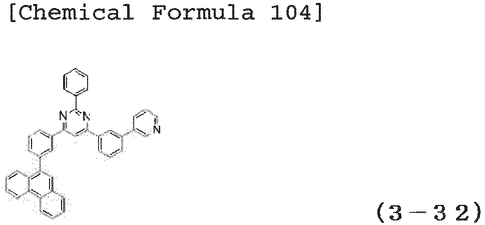

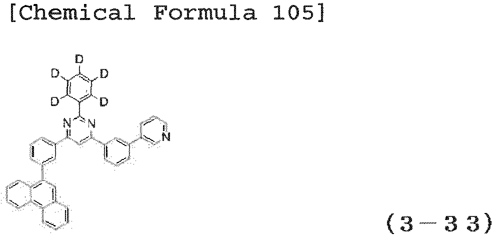

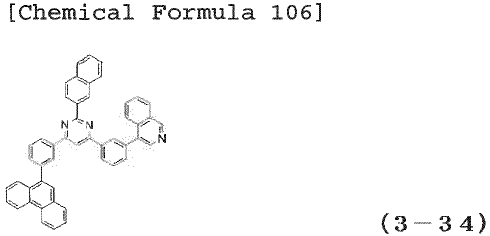

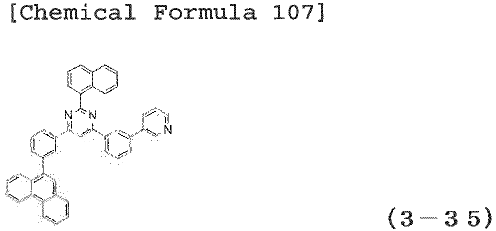

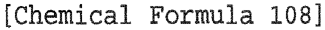

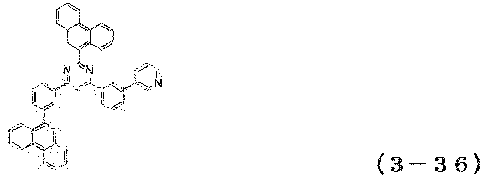

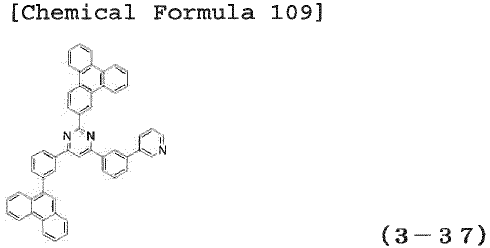

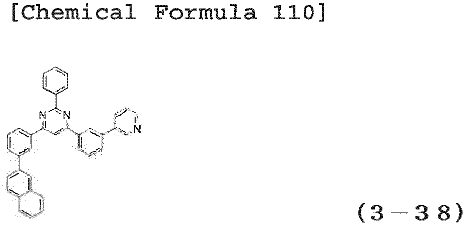

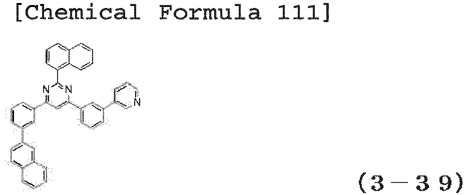

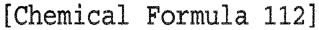

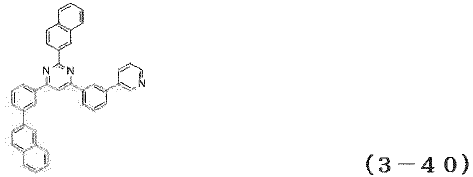

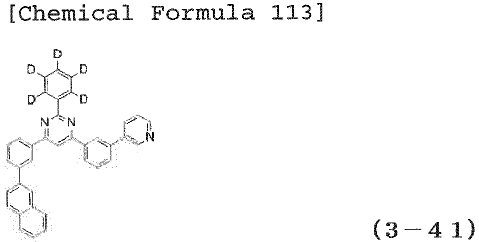

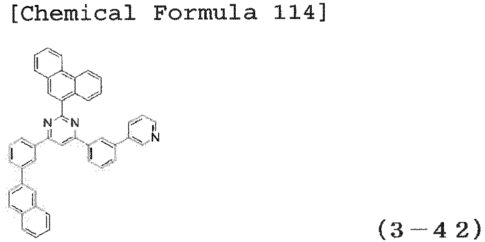

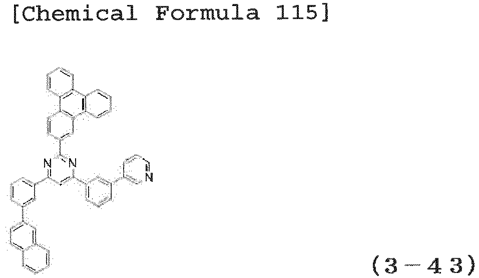

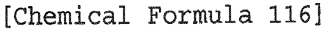

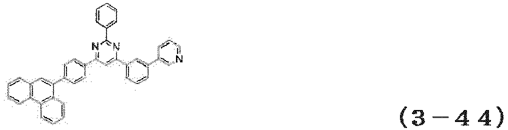

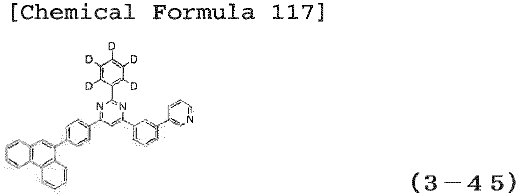

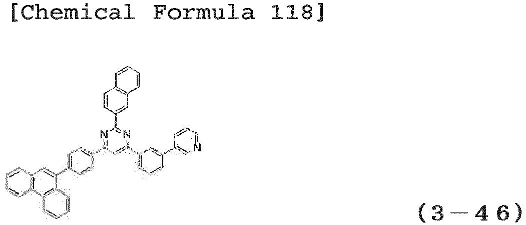

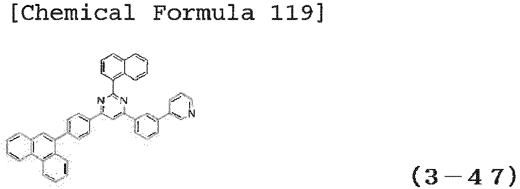

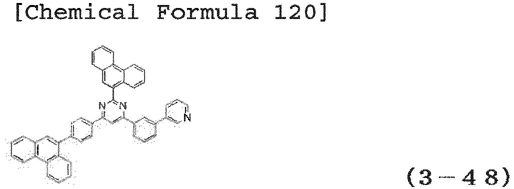

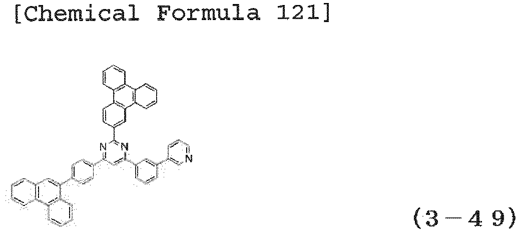

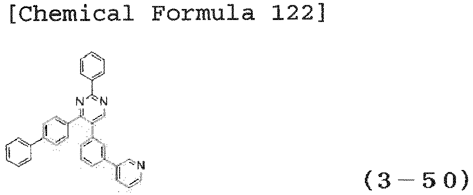

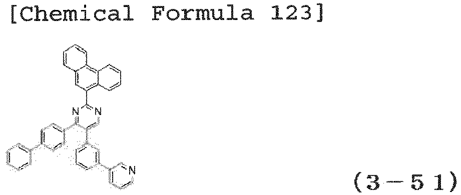

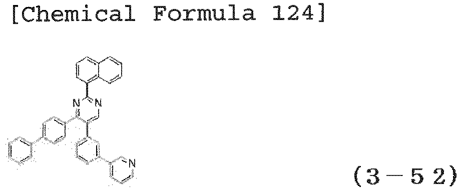

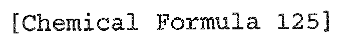

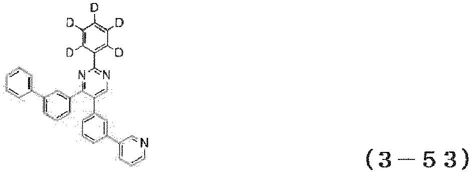

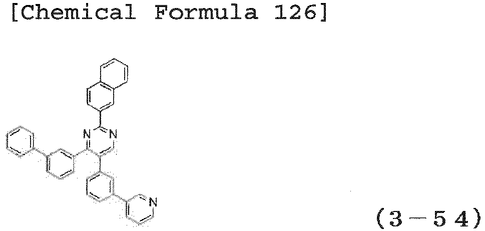

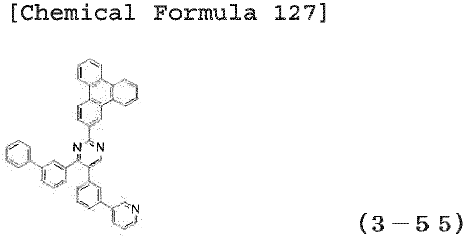

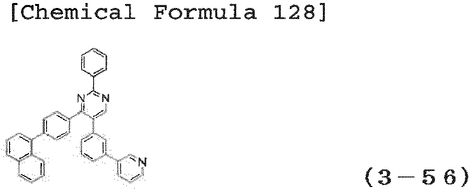

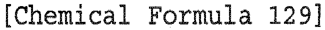

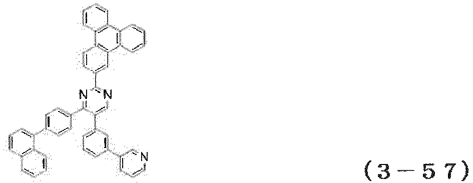

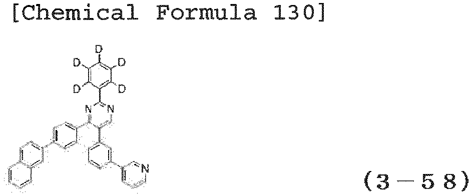

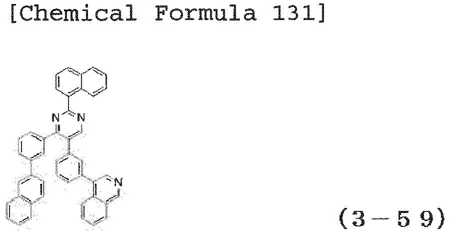

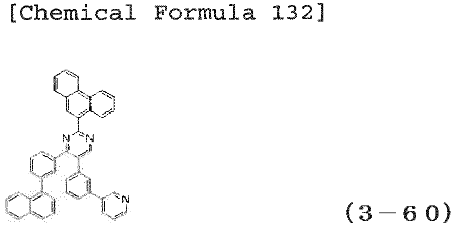

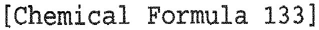

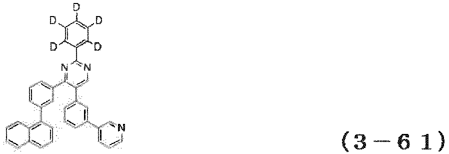

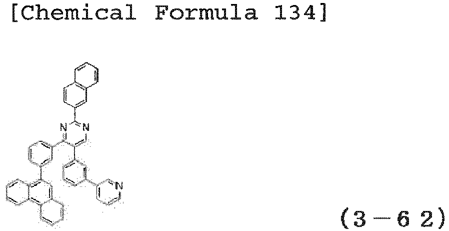

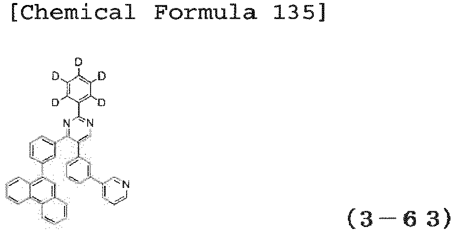

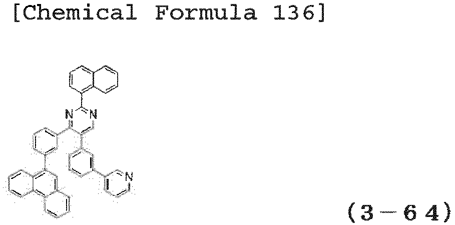

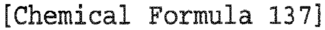

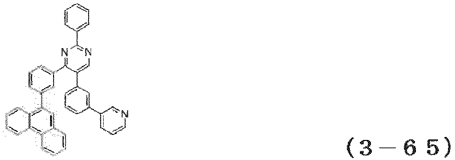

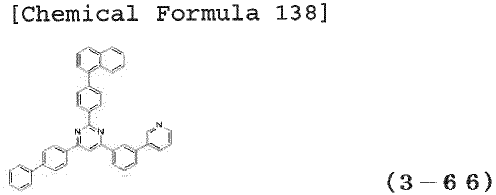

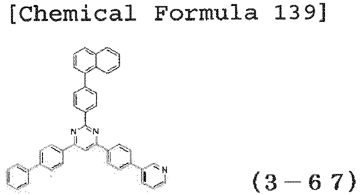

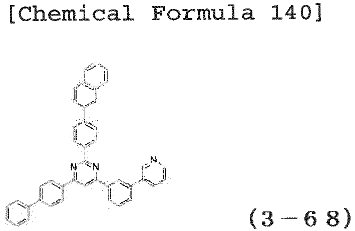

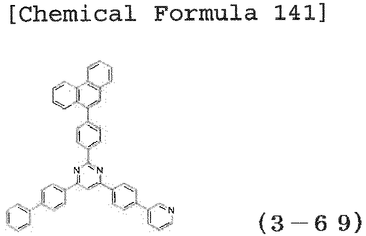

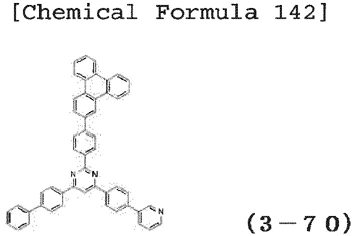

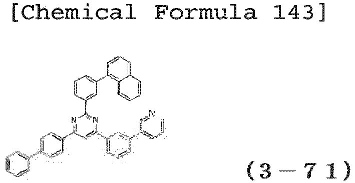

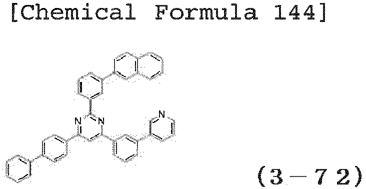

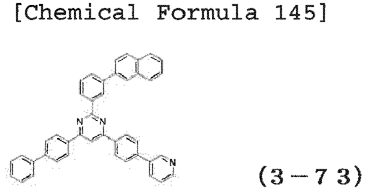

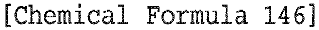

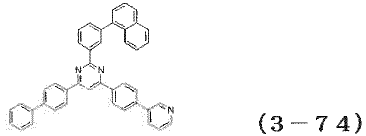

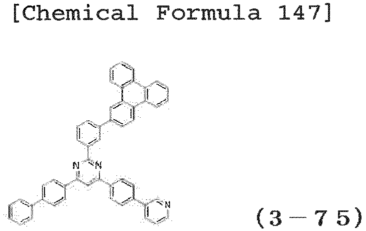

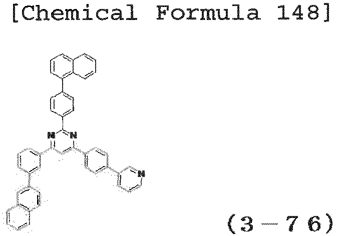

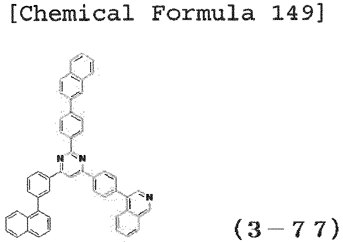

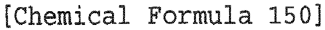

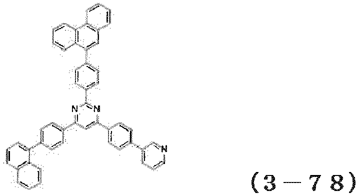

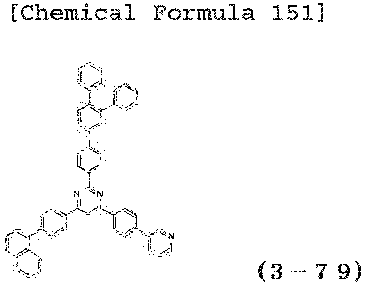

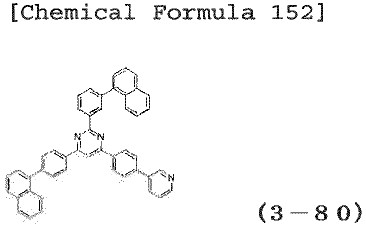

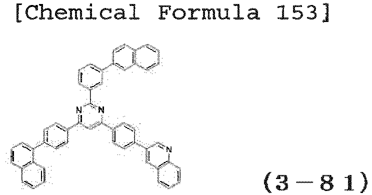

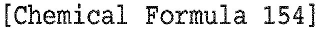

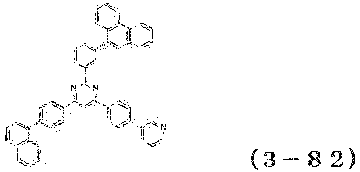

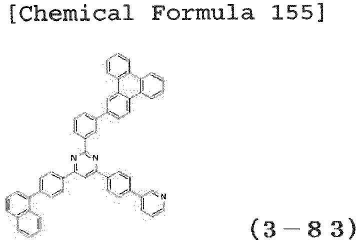

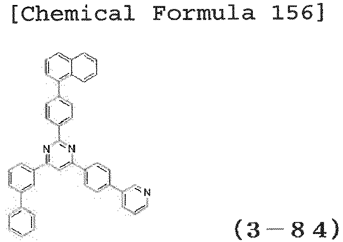

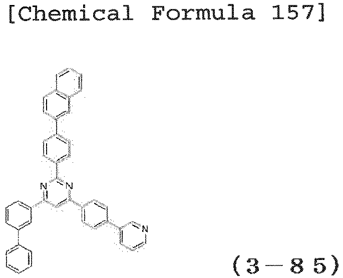

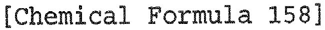

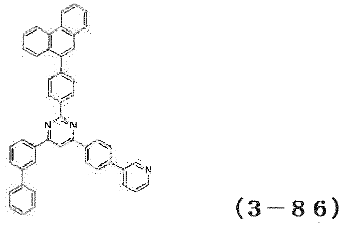

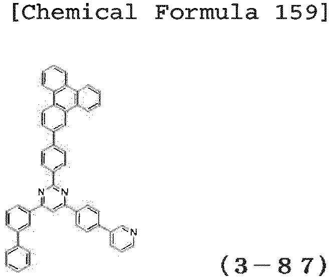

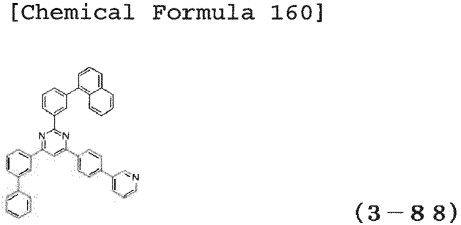

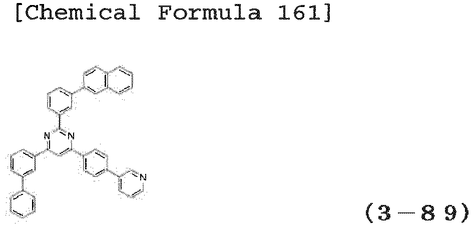

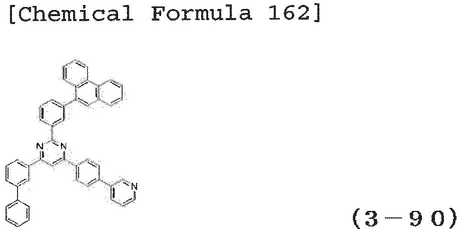

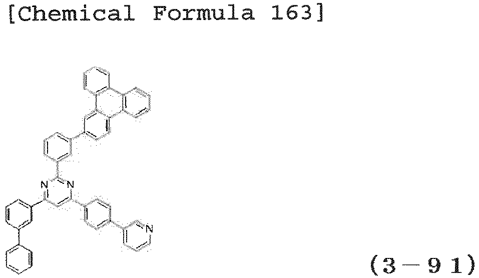

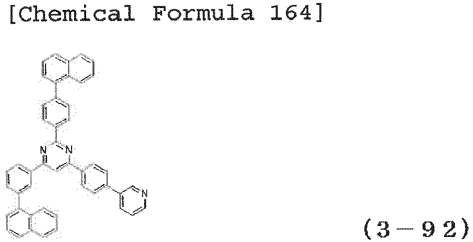

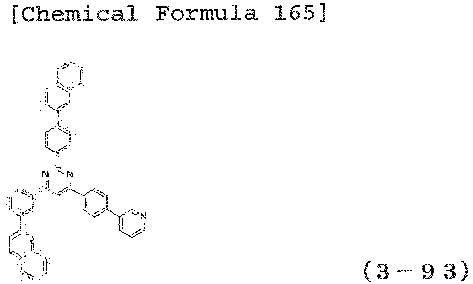

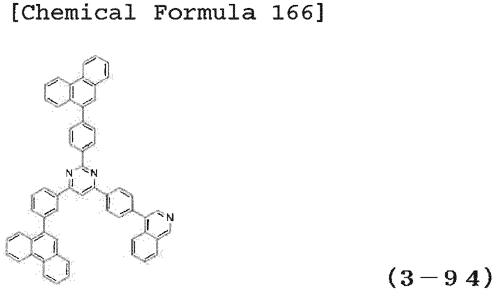

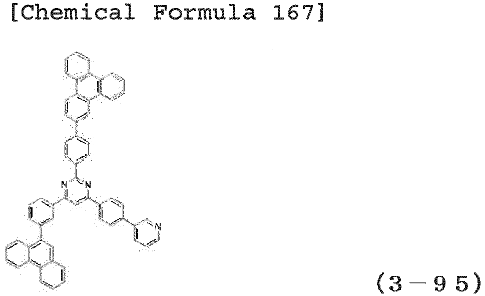

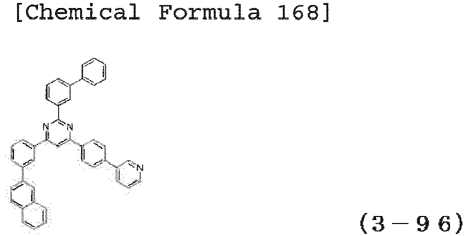

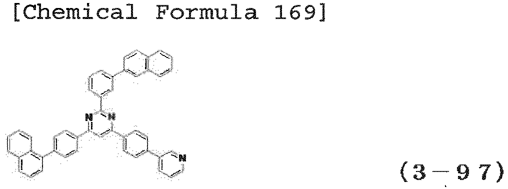

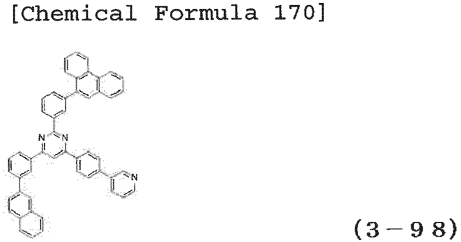

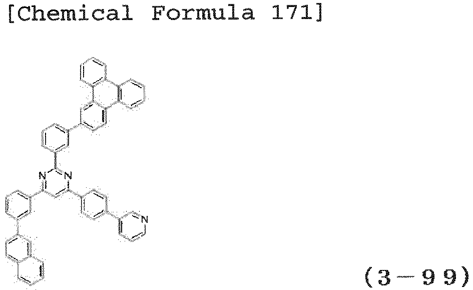

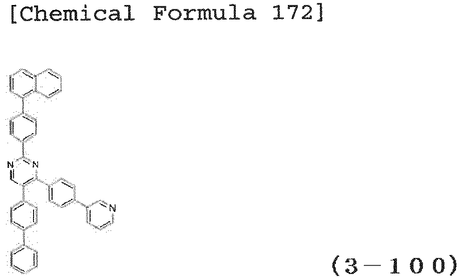

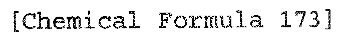

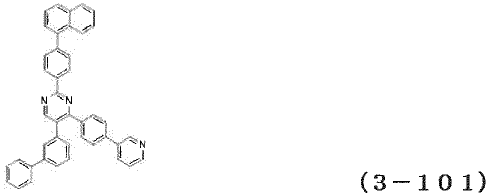

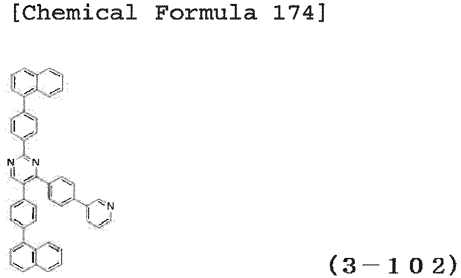

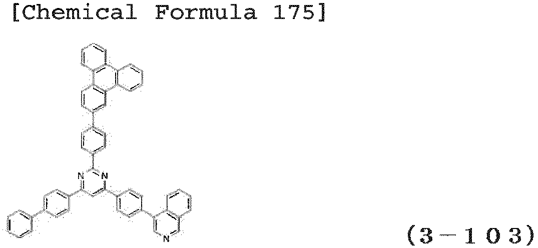

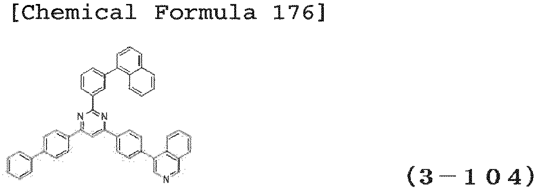

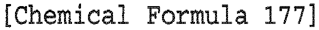

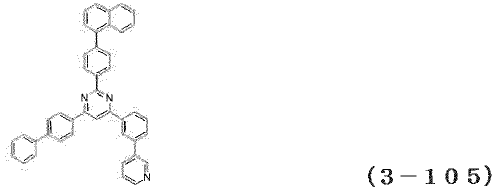

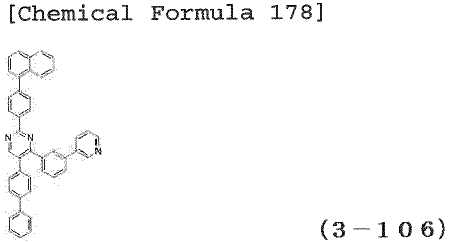

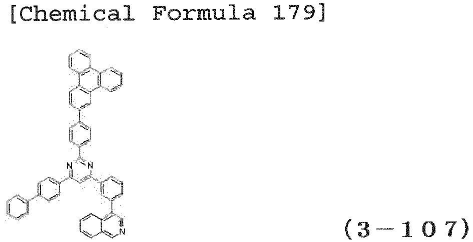

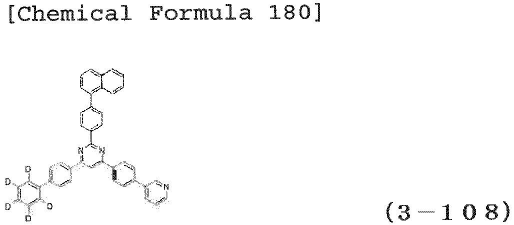

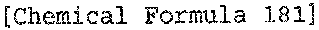

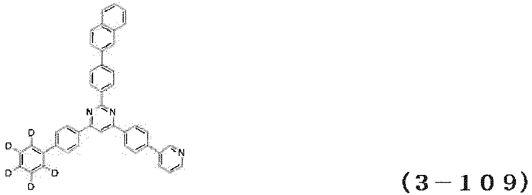

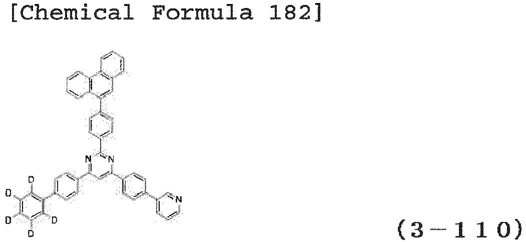

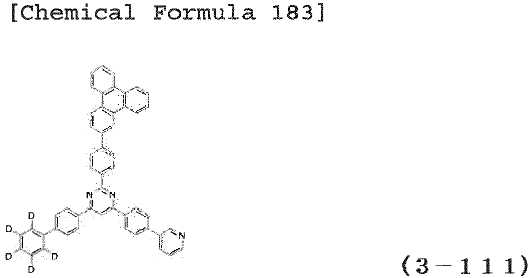

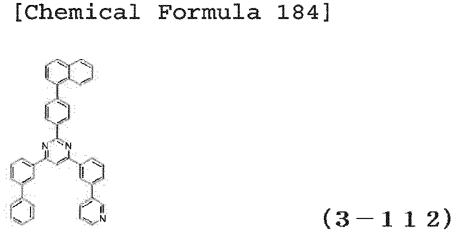

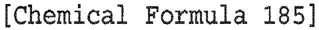

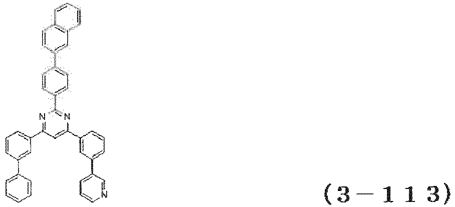

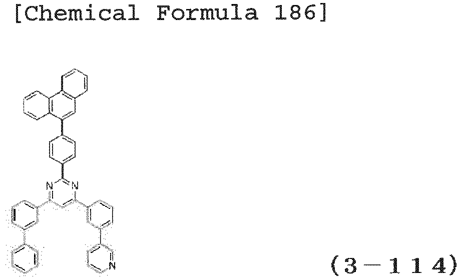

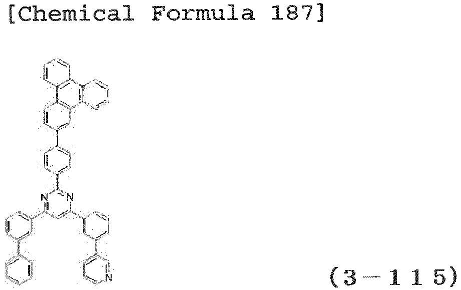

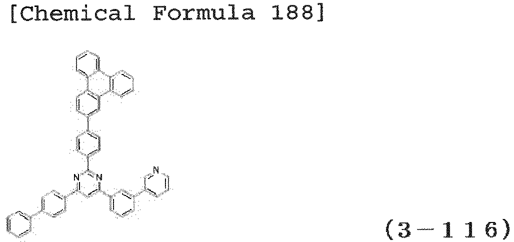

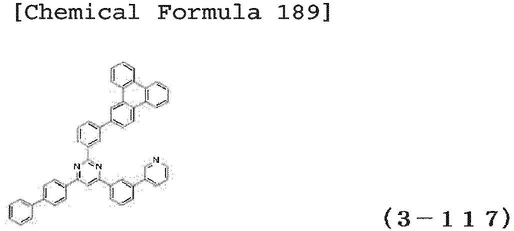

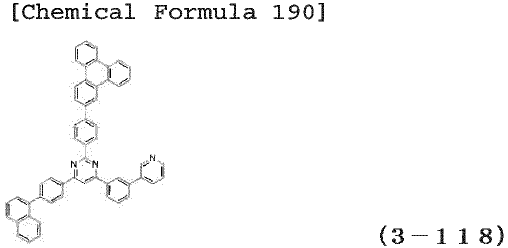

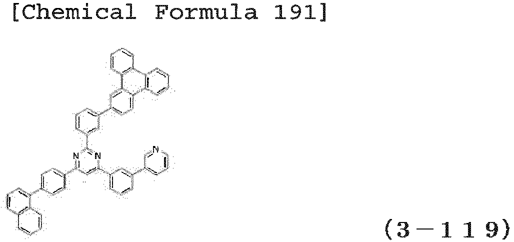

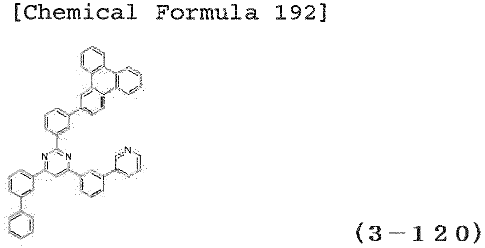

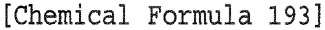

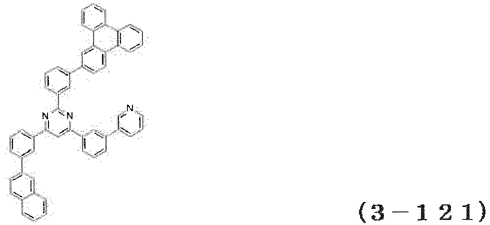

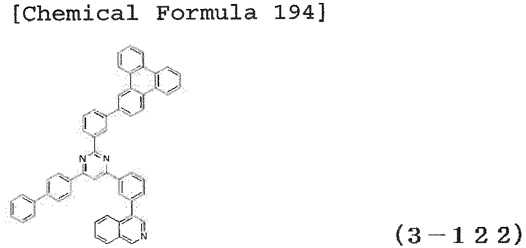

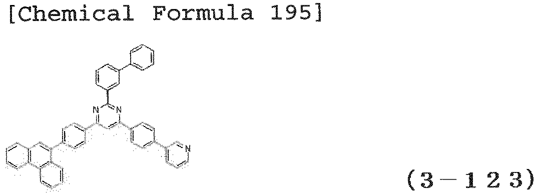

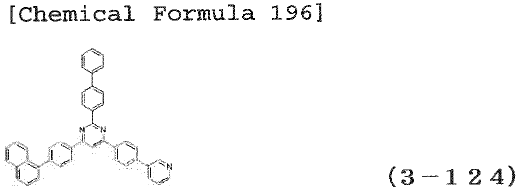

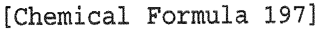

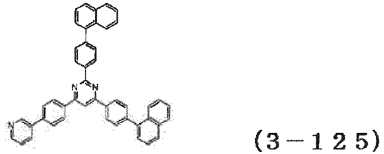

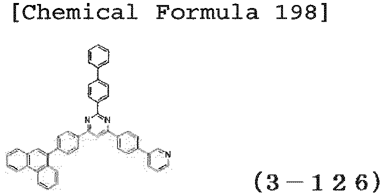

The compounds described above having a pyrimidine ring structure can be synthesized by a known method (refer to PTL 7, for example).

The arylamine compounds of the general formula (1) and the general formula (1a) were purified by methods such as column chromatography, adsorption using, for example, a silica gel, activated carbon, or activated clay, recrystallization or crystallization using a solvent, and a sublimation purification method. The compounds were identified by an NMR analysis. A melting point, a glass transition point (Tg), and a work function were measured as material property values. The melting point can be used as an index of vapor deposition, the glass transition point (Tg) as an index of stability in a thin-film state, and the work function as an index of hole transportability and hole blocking performance.

Other compounds used for the organic EL device of the present invention were purified by methods such as column chromatography, adsorption using, for example, a silica gel, activated carbon, or activated clay, recrystallization or crystallization using a solvent, and a sublimation purification method, and finally purified by a sublimation purification method.

The melting point and the glass transition point (Tg) were measured by a high-sensitive differential scanning calorimeter (DSC3100SA produced by Bruker AXS) using powder.

For the measurement of the work function, a 100 nm-thick thin film was fabricated on an ITO substrate, and an ionization potential measuring device (PYS-202 produced by Sumitomo Heavy Industries, Ltd.) was used.

The organic EL device of the present invention may have a structure including an anode, a hole injection layer, a hole transport layer, a light emitting layer, an electron transport layer, and a cathode successively formed on a substrate, optionally with an electron blocking layer between the hole transport layer and the light emitting layer, a hole blocking layer between the light emitting layer and the electron transport layer, and an electron injection layer between the electron transport layer and the cathode. Some of the organic layers in the multilayer structure may be omitted, or may serve more than one function. For example, a single organic layer may serve as the hole injection layer and the hole transport layer, or as the electron injection layer and the electron transport layer, and so on. Further, any of the layers may be configured to laminate two or more organic layers having the same function, and the hole transport layer may have a two-layer laminated structure, the light emitting layer may have a two-layer laminated structure, the electron transport layer may have a two-layer laminated structure, and so on. The organic EL device of the present invention is preferably configured such that the hole transport layer has a two-layer laminated structure of a first hole transport layer and a second hole transport layer.

Electrode materials with high work functions such as ITO and gold are used as the anode of the organic EL device of the present invention. The hole injection layer of the organic EL device of the present invention may be made of, for example, material such as starburst-type triphenylamine derivatives and various triphenylamine tetramers; porphyrin compounds as represented by copper phthalocyanine; accepting heterocyclic compounds such as hexacyano azatriphenylene; and coating-type polymer materials, in addition to the arylamine compounds of the general formula (1). These materials may be formed into a thin film by a vapor deposition method or other known methods such as a spin coating method and an inkjet method.

The arylamine compounds of the general formula (1) are used as the hole transport layer of the organic EL device of the present invention. These may be individually deposited for film forming, may be used as a single layer deposited mixed with other hole transporting materials, or may be formed as a laminate of individually deposited layers, a laminate of mixedly deposited layers, or a laminate of the individually deposited layer and the mixedly deposited layer. These materials may be formed into a thin-film by a vapor deposition method or other known methods such as a spin coating method and an inkjet method.

Examples of a hole transporting material that can be mixed or can be used at the same time with the arylamine compounds of the general formula (1) can be benzidine derivatives such as N,N'-diphenyl-N,N'-di(m-tolyl)benzidine (TPD), N,N'-diphenyl-N,N'-di(α-naphthyl)benzidine (NPD), and N,N,N',N'-tetrabiphenylylbenzidine; 1,1-bis[4-(di-4-tolylamino)phenyl]cyclohexane (TAPC); triphenylamine derivatives having two triphenylamine skeletons as a whole molecule; triphenylamine derivatives having four triphenylamine skeletons as a whole molecule; and triphenylamine derivatives having three triphenylamine skeletons as a whole molecule.

The material used for the hole injection layer or the hole transport layer may be obtained by p-doping materials such as trisbromophenylamine hexachloroantimony, and radialene derivatives (refer to WO2014/009310, for example) into a material commonly used for these layers, or may be, for example, polymer compounds each having, as a part of the compound structure, a structure of a benzidine derivative such as TPD.

Examples of material used for the electron blocking layer of the organic EL device of the present invention can be compounds having an electron blocking effect, including, for example, carbazole derivatives such as 4,4',4"-tri(N-carbazolyl)triphenylamine (TCTA), 9,9-bis[4-(carbazol-9-yl)phenyl]fluorene, 1,3-bis(carbazol-9-yl)benzene (mCP), and 2,2-bis(4-carbazol-9-ylphenyl)adamantane (Ad-Cz); and compounds having a triphenylsilyl group and a triarylamine structure, as represented by 9-[4-(carbazol-9-yl)phenyl]-9-[4-(triphenylsilyl)phenyl]-9H-fluorene, in addition to the arylamine compounds of the general formula (1). These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin-film by using a vapor deposition method or other known methods such as a spin coating method and an inkjet method.

Examples of material used for the light emitting layer of the organic EL device of the present invention can be various metal complexes such as quinolinol derivative metal complexes including Alq₃, anthracene derivatives, bis(styryl)benzene derivatives, oxazole derivatives, and polyparaphenylene vinylene derivatives, in addition to the amine derivative having a condensed ring structure of the following general formula (2) and the pyrene derivative. Further, the light emitting layer may be made of a host material and a dopant material. Examples of the host material can be thiazole derivatives, benzimidazole derivatives, and polydialkyl fluorene derivatives, in addition to the above light-emitting materials. Examples of the dopant material can be quinacridone, coumarin, rubrene, perylene, derivatives thereof, benzopyran derivatives, indenophenanthrene derivatives, rhodamine derivatives, and aminostyryl derivatives, in addition to the amine derivative having a condensed ring structure of the following general formula (2) and the pyrene derivative. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer.

The dopant material in the light emitting layer of the organic EL device of the present invention is preferably the amine derivative having a condensed ring structure of the general formula (2) and the pyrene derivative, far preferably, the amine derivative having a condensed ring structure of the general formula (2).

Further, the light-emitting material may be a phosphorescent material. Phosphorescent materials as metal complexes of metals such as iridium and platinum may be used. Examples of the phosphorescent materials include green phosphorescent materials such as Ir(ppy)₃, blue phosphorescent materials such as FIrpic and FIr6, and red phosphorescent materials such as Btp₂Ir(acac). Here, carbazole derivatives such as 4,4'-di(N-carbazolyl)biphenyl (CBP), TCTA, and mCP may be used as the hole injecting and transporting host material. Compounds such as p-bis(triphenylsilyl)benzene (UGH2) and 2,2',2"-(1,3,5-phenylene)-tris(1-phenyl-1H-benzimidazole) (TPBI) may be used as the electron transporting host material. In this way, a high-performance organic EL device can be produced.

In order to avoid concentration quenching, the doping of the host material with the phosphorescent light-emitting material should preferably be made by coevaporation in a range of 1 to 30 weight percent with respect to the whole light emitting layer.

Further, Examples of the light-emitting material may be delayed fluorescent-emitting material such as a CDCB derivative of PIC-TRZ, CC2TA, PXZ-TRZ, 4CzIPN or the like (refer to NPL 3, for example).

These materials may be formed into a thin-film by using a vapor deposition method or other known methods such as a spin coating method and an inkjet method.

The hole blocking layer of the organic EL device of the present invention may be formed by using hole blocking compounds such as various rare earth complexes, triazole derivatives, triazine derivatives, and oxadiazole derivatives, in addition to the metal complexes of phenanthroline derivatives such as bathocuproin (BCP), and the metal complexes of quinolinol derivatives such as aluminum(III) bis(2-methyl-8-quinolinate)-4-phenylphenolate (hereinafter referred to as BAlq). These materials may also serve as the material of the electron transport layer. These may be individually deposited for film forming, may be used as a single layer deposited mixed with other materials, or may be formed as a laminate of individually deposited layers, a laminate of mixedly deposited layers, or a laminate of the individually deposited layer and the mixedly deposited layer. These materials may be formed into a thin-film by using a vapor deposition method or other known methods such as a spin coating method and an inkjet method.

Material used for the electron transport layer of the organic EL device of the present invention can be preferably the compounds of the general formula (3) having a pyrimidine ring structure. These may be individually deposited for film forming, may be used as a single layer deposited mixed with other electron transport materials, or may be formed as a laminate of individually deposited layers, a laminate of mixedly deposited layers, or a laminate of the individually deposited layer and the mixedly deposited layer. These materials may be formed into a thin-film by using a vapor deposition method or other known methods such as a spin coating method and an inkjet method.

Examples of the electron transporting material that can be mixed or can be used at the same time with the compounds of the general formula (3) having a pyrimidine ring structure can be metal complexes of quinolinol derivatives including Alq₃ and BAlq, various metal complexes, triazole derivatives, triazine derivatives, oxadiazole derivatives, pyridine derivatives, pyrimidine derivatives, benzimidazole derivatives, thiadiazole derivatives, anthracene derivatives, carbodiimide derivatives, quinoxaline derivatives, pyridoindole derivatives, phenanthroline derivatives, and silole derivatives.

Examples of material used for the electron injection layer of the organic EL device of the present invention can be alkali metal salts such as lithium fluoride and cesium fluoride; alkaline earth metal salts such as magnesium fluoride; and metal oxides such as aluminum oxide. However, the electron injection layer may be omitted in the preferred selection of the electron transport layer and the cathode.

The cathode of the organic EL device of the present invention may be made of an electrode material with a low work function such as aluminum, or an alloy of an electrode material with an even lower work function such as a magnesium-silver alloy, a magnesium-indium alloy, or an aluminum-magnesium alloy.

The following describes an embodiment of the present invention in more detail based on Examples. The present invention, however, is not restricted to the following Examples.

### Example 1

### <Synthesis of 4-bis(biphenyl-4-yl)amino-4'-{(biphenyl-4-yl)-phenylamino}-2-phenyl-biphenyl (Compound 1-1)>

bis(biphenyl-4-yl)-(6-bromobiphenyl-3- yl)amine (10.0 g), 4-{(biphenyl-4-yl)-phenylamino} phenylboronicacid (7.9 g), tetrakistriphenylphosphine palladium (0) (0.60 g), potassium carbonate (5.0 g), toluene (80 mL), ethanol (40 mL), and water (30 mL) were added into a nitrogen-substituted reaction vessel. The mixture was heated, and stirred at 100°C for overnight. After cooling, an organic layer was collected by liquid separation. The organic layer was concentrated, and then purified by column chromatography (support: silica gel, eluent: dichloromethane / heptane), whereby a white powder of 4-bis(biphenyl-4-yl)amino-4'-{(biphenyl-4-yl)-phenylamino}-2-phenyl-biphenyl (Compound 1-1; 5.30 g; yield: 37%) was obtained.

The structure of the obtained white powder was identified by NMR.

¹H-NMR (CDCl₃) detected 44 hydrogen signals, as follows.

δ(ppm) = 7.65-7.60 (5H), 7.59-7.53 (5H), 7.52-7.40 (9H), 7.39-7.21 (15H), 7.20-7.10 (5H), 7.90-6.91 (5H).

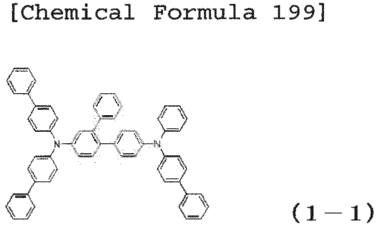

### Example 2

### <Synthesis of 4-{(biphenyl-4-yl)-(4-naphthalene-1-yl-phenyl)amino}-4'-{(biphenyl-4-yl)-phenylamino}-2-phenyl-biphenyl (Compound 1-3)>

The reaction was carried out under the same conditions as those of Example 1, except that bis(biphenyl-4-yl)-(6-bromobiphenyl-3-yl)amine was replaced with (biphenyl-4-yl)-(6-bromobiphenyl-3-yl)-(4-naphthalene-1-yl-phenyl)amine, whereby a whitish powder of 4-{(biphenyl-4-yl)-(4-naphthalene-1-yl-phenyl)amino}-4'-{(biphenyl-4-yl)-phenylamino}-2-phenyl-biphenyl (Compound 1-3; 9.70 g; yield: 69%) was obtained.

The structure of the obtained whitish powder was identified by NMR.

¹H-NMR (CDCl₃) detected 46 hydrogen signals, as follows.

5(ppm) = 8.07 (1H), 7.93 (1H), 7.87 (1H), 7.67-7.54 (7H), 7.54-7.11 (31H), 7.69-6.92 (5H).

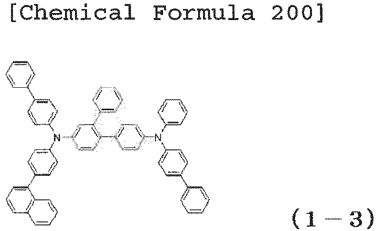

### Example 3

### <Synthesis of 4-{(biphenyl-4-yl)-(p-terphenyl-4-yl)amino}-4'-{(biphenyl-4-yl)-phenylamino}-2-phenyl-biphenyl (Compound 1-5)>

The reaction was carried out under the same conditions as those of Example 1, except that bis(biphenyl-4-yl)-(6-bromobiphenyl-3-yl)amine was replaced with (biphenyl-4-yl)-(6-bromobiphenyl-3-yl)-(p-terphenyl-4-yl)amine, whereby a white powder of 4-{(biphenyl-4-yl)-(p-terphenyl-4-yl)amino}-4'-{(biphenyl-4-yl)-phenylamino}-2-phenyl-biphenyl (Compound 1-5; 6.76 g; yield: 57%) was obtained.

The structure of the obtained white powder was identified by NMR.

¹H-NMR (CDCl₃) detected 48 hydrogen signals, as follows.

5(ppm) = 7.71-7.10 (43H), 7.08-6.93 (5H).

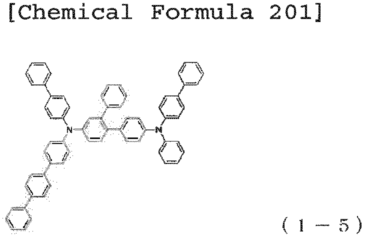

### Example 4

### <Synthesis of 4-{(biphenyl-4-yl)-phenylamino}-4'-{bis(4-naphthalene-1-yl-phenyl)amino}-2'-phenyl-biphenyl (Compound 1-6)>

The reaction was carried out under the same conditions as those of Example 1, except that bis(biphenyl-4-yl)-(6-bromobiphenyl-3-yl)amine was replaced with bis(4-naphthalene-1-yl-phenyl)-(6-bromobiphenyl-3-yl)amine, whereby a yellowish white powder of 4-{(biphenyl-4-yl)-phenylamino}-4'-{bis(4-naphthalene-1-yl-phenyl)amino}-2'-phenyl-biphenyl (Compound 1-6; 10.0 g; yield: 73%) was obtained.

The structure of the obtained yellowish white powder was identified by NMR.

¹H-NMR (CDCl₃) detected 48 hydrogen signals, as follows.

5(ppm) = 8.08 (1H), 7.94 (1H), 7.88 (1H), 7.63-7.20 (40H), 7.19-6.92 (5H).

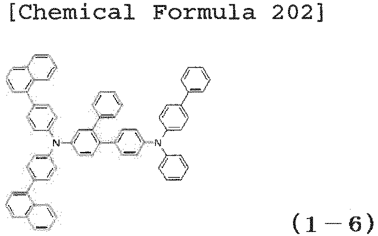

### Example 5

### <Synthesis of 4-{(9,9-dimethylfluoren-2-yl)-(biphenyl-4-yl)amino}-4'-(biphenyl-4-yl-phenylamino)-2-phenyl-biphenyl (Compound 1-7)>

The reaction was carried out under the same conditions as those of Example 1, except that bis(biphenyl-4-yl)-(6-bromobiphenyl-3-yl)amine was replaced with (9,9-dimethylfluoren-2-yl)-(biphenyl-4-yl)-(6-bromobiphenyl-3-yl)amine, whereby a white powder of 4-{(9,9-dimethylfluoren-2-yl)-(biphenyl-4-yl)amino}-4'-(biphenyl-4-yl-phenylamino)-2-phenyl-biphenyl (Compound 1-7; 8.30 g; yield: 49%) was obtained.

The structure of the obtained white powder was identified by NMR.

¹H-NMR (CDCl₃) detected 48 hydrogen signals, as follows.

δ(ppm) = 7.72-7.60 (2H), 7.59-7.52 (2H), 7.51-7.10 (35H), 7.09-6.90 (3H), 1.56 (6H).

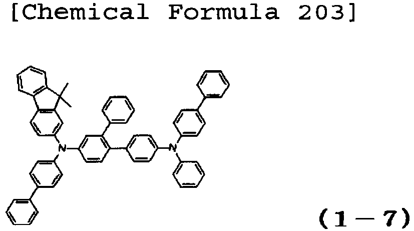

### Example 6

### <Synthesis of 3-{(9,9-dimethylfluoren-2-yl)-(biphenyl-4-yl)amino}-6-(9-phenylcarbazol-3-yl)-biphenyl (Compound 1-8)>

The reaction was carried out under the same conditions as those of Example 1, except that bis(biphenyl-4-yl)-(6-bromobiphenyl-3-yl)amine was replaced with (9,9-dimethylfluoren-2-yl)-(biphenyl-4-yl)-(6-bromobiphenyl-3-yl)amine, and 4-{(biphenyl-4-yl)-phenylamino} phenylboronicacid was replaced with (9-phenylcarbazol-3-yl) boronicacid, whereby a white powder of 3-{(9,9-dimethylfluoren-2-yl)-(biphenyl-4-yl)amino)-6-(9-phenylcarbazol-3-yl)-biphenyl (Compound 1-8; 17.4 g; yield: 85%) was obtained.

The structure of the obtained white powder was identified by NMR.

¹H-NMR (CDCl₃) detected 42 hydrogen signals, as follows.

δ(ppm) = 8.05 (2H), 7.72-7.10 (34H), 1.52 (6H).

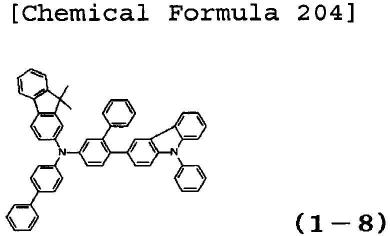

### Example 7

### <Synthesis of 4-{(naphthalene-2-yl)phenyl-4-yl}-(biphenyl-4-yl)amino-4'-{(biphenyl-4-yl)-phenylamino}-2-phenyl-1,1'-biphenyl (Compound 1-4)>

The reaction was carried out under the same conditions as those of Example 1, except that bis(biphenyl-4-yl)-(6-bromobiphenyl-3-yl)amine was replaced with (6-bromo-1,1'-biphenyl-3-yl)-{(naphthalene-2-yl)phenyl-4-yl}-(biphenyl-4-yl)amine, whereby a white powder of 4-{(naphthalene-2-yl)phenyl-4-yl}-(biphenyl-4-yl)amino-4'-{(biphenyl-4-yl)-phenylamino}-2-phenyl-1,1'-biphenyl (Compound 1-4; 6.1 g; yield: 58%) was obtained.

The structure of the obtained white powder was identified by NMR.

¹H-NMR (CDCl₃) detected 46 hydrogen signals, as follows.

δ(ppm) = 8.07 (1H), 7.95-7.76 (4H), 7.68-6.98 (41H).

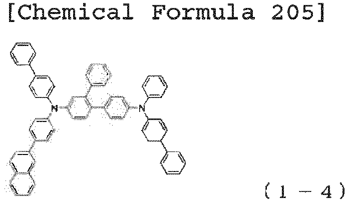

### Example 8

### <Synthesis of 4,4"-bis{(biphenyl-4-yl)-phenylamino}-2-phenyl-1,1';4',1"-terphenyl (Compound 1-19)>

The reaction was carried out under the same conditions as those of Example 1, except that bis(biphenyl-4-yl)-(6-bromobiphenyl-3-yl)amine was replaced with (6-bromo-1,1'-biphenyl-3-yl)-(1,1'-biphenyl-4-yl)phenylamine, whereby a white powder of 4,4"-bis{(biphenyl-4-yl)-phenylamino}-2-phenyl-1,1';4',1"-terphenyl (Compound 1-19; 12.9 g; yield: 43%) was obtained.

The structure of the obtained white powder was identified by NMR.

¹H-NMR (CDCl₃) detected 44 hydrogen signals, as follows.

δ(ppm) = 7.65-7.61 (4H), 7.57-7.07 (40H).

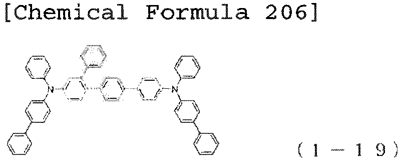

Reference Example 9 does not forming part of the present invention.

### <Synthesis of 4,4"-bis{(biphenyl-4-yl)-phenylamino}-3,3"-diphenyl-1,1';4',1"'-terphenyl (Compound 1-27)>

4,4"-bis{(biphenyl-4-yl)-amino}-3,3"-diphenyl-1,1':4',1"-terphenyl (16.3 g), iodobenzene (18.6 g), copper powder (0.29 g), potassium carbonate (9.61 g), 3,5-di-tert-butylsalicylicacid (1.85 g), sodium hydrogensulfite (0.47 g), dodecylbenzene (20 mL) were added into a nitrogen-substituted reaction vessel. The mixture was heated and stirred at 190 to 200 °C for 17 hours. The mixture was cooled, toluene (1500 mL), a silica gel (40 g), and activated clay (20 g) was added thereto, and stirred. After the insoluble matter was removed by filtration, the filtrate was concentrated. The crude product was purified by recrystallization with chlorobenzene, the recrystallization procedure was repeated to obtain a white powder of 4,4"-bis{(biphenyl-4-yl)-phenylamino}-3,3"-diphenyl-1,1':4',1"-terphenyl (Compound 1-27; 9.65 g; yield 49%).

The structure of the obtained white powder was identified by NMR.

¹H-NMR (CDCl₃) detected 48 hydrogen signals, as follows.

δ(ppm) = 7.62 (4H), 7.52 (4H), 7.45 (4H), 7.36-7.04 (32H), 6.99 (4H).

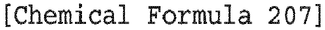

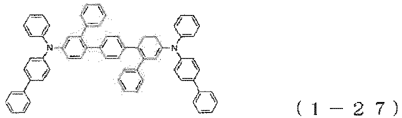

### Example 10

### <Synthesis of 4,4"-bis{(biphenyl-4-yl)-phenylamino}-3-phenyl-1,1';3',1"-terphenyl (Compound 1-30) >

4-{(biphenyl-4-yl)-phenylamino}-4"-{(biphenyl-4-yl)-amino}-3-phenyl-1,1':3',1"-terphenyl (17.0 g), bromobenzene (4.12 g), palladium acetate (0.13 g), a toluene solution (0.33 mL) containing 50% (w/v) tri-tert-butylphosphine, sodium tert-butoxide (2.73 g), and toluene (190 mL) were added into a nitrogen-substituted reaction vessel. The mixture was heated and stirred at 80°C for 3 hours. After cooling, the insoluble matter was removed by filtration, and the filtrate was concentrated. The crude product was purified by column chromatography (support: silica gel, eluent: toluene / n-hexane), a solid precipitated by adding acetone was collected, whereby a white powder of 4,4"-bis{(biphenyl-4-yl)-phenylamino}-3-phenyl-1,1':3',1"-terphenyl (Compound 1-30; 13.29 g; yield: 71%) was obtained.

The structure of the obtained white powder was identified by NMR.

¹H-NMR (CDCl₃) detected 44 hydrogen signals, as follows.

δ(ppm) = 7.62-7.58 (4H), 7.55-7.49 (4H), 7.48-7.38 (6H), 7.37-7.05 (30H).

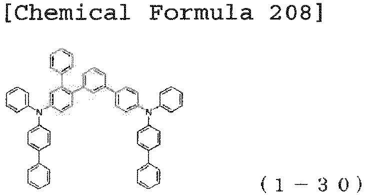

### Example 11

### <Synthesis of 4-bis(biphenyl-4-yl)amino-4'-{(biphenyl-4-yl)-phenylamino}-2,6-diphenyl-biphenyl (Compound 1-41)>

The reaction was carried out under the same conditions as those of Example 1, except that bis(biphenyl-4-yl)-(6-bromobiphenyl-3-yl)amine was replaced with 4-bis(biphenyl-4-yl) amino-2,6- diphenyl-bromobenzene, whereby a white powder of 4-bis(biphenyl-4-yl)amino-4'-{(biphenyl-4-yl)-phenylamino}-2,6-diphenyl-biphenyl (Compound 1-41; 12.7 g; yield: 57%) was obtained.

The structure of the obtained white powder was identified by NMR.

¹H-NMR (CDCl₃) detected 48 hydrogen signals, as follows.

δ(ppm) = 7.65-7.53 (8H), 7.48-6.97 (36H), 6.79-6.73 (4H) .

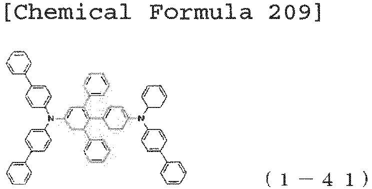

### Example 12

The melting points and the glass transition points of the arylamine compounds of the general formula (1) were measured using a high-sensitive differential scanning calorimeter (DSC3100SA produced by Bruker AXS).

| | Melting point | Glass transition point |
|---|---|---|
| Compound of Example 1 | No point melting observed | 118 °C |
| Compound of Example 2 | No point melting observed | 121 °C |
| Compound of Example 3 | No point melting observed | 125 °C |
| Compound of Example 4 | No point melting observed | 125 °C |
| Compound of Example 5 | No point melting observed | 125 °C |
| Compound of Example 6 | No point melting observed | 139 °C |
| Compound of Example 7 | No point melting observed | 121 °C |
| Compound of Example 8 | No point melting observed | 120 °C |
| Compound of Reference Example 9 | 263 °C | 124 °C |
| Compound of Example 10 | No point melting observed | 117 °C |
| Compound of Example 11 | 238 °C | 126 °C |

The arylamine compounds of the general formula (1) have glass transition points of 100°C or higher, demonstrating that the compounds have a stable thin-film state.

### Example 13

A 100 nm-thick vapor-deposited film was fabricated on an ITO substrate using the arylamine compounds of the general formula (1), and a work function was measured using an ionization potential measuring device (PYS-202 produced by Sumitomo Heavy Industries, Ltd.).

| | Work function |
|---|---|
| Compound of Example 1 | 5.63 eV |
| Compound of Example 2 | 5.62 eV |
| Compound of Example 3 | 5.62 eV |
| Compound of Example 4 | 5.65 eV |
| Compound of Example 5 | 5.57 eV |
| Compound of Example 6 | 5.56 eV |
| Compound of Example 7 | 5.60 eV |
| Compound of Example 8 | 5.70 eV |
| Compound of Reference Example 9 | 5.74 eV |
| Compound of Example 10 | 5.79 eV |
| Compound of Example 11 | 5.67 eV |

As the results show, the arylamine compounds of the general formula (1) have desirable energy levels compared to the work function 5.4 eV of common hole transport materials such as NPD and TPD, and thus possess desirable hole transportability.

### Example 14

The organic EL device, as shown in FIG. 1, was fabricated by vapor-depositing a hole injection layer 3, a hole transport layer 4, a light emitting layer 5, an electron transport layer 6, an electron injection layer 7, and a cathode (aluminum electrode) 8 in this order on a glass substrate 1 on which an ITO electrode was formed as a transparent anode 2 beforehand.

Specifically, the glass substrate 1 having ITO having a film thickness of 150 nm formed thereon was subjected to ultrasonic washing in isopropyl alcohol for 20 minutes and then dried for 10 minutes on a hot plate heated to 200°C. Thereafter, after performing a UV ozone treatment for 15 minutes, the glass substrate 1 with ITO was installed in a vacuum vapor deposition apparatus, and the pressure was reduced to 0.001 Pa or lower. Subsequently, as the hole injection layer 3 covering the transparent anode 2, Compound (HIM-1) of the structural formula below were formed in a film thickness of 5 nm. As the hole transport layer 4 on the hole injection layer 3, Compound (1-7) of Example 5 was formed in a film thickness of 65 nm. As the light emitting layer 5 on the hole transport layer 4, Compound (EMD-1) of the structural formula below and Compound (EMH-1) of the structural formula below were formed in a film thickness of 20 nm by dual vapor deposition at a vapor deposition rate that satisfies a vapor deposition rate ratio of EMD-1/EMH-1 = 5/95. As the electron transport layer 6 on the light emitting layer 5, Compound (3-125) of the structural formula below and Compound (ETM-1) of the structural formula below were formed in a film thickness of 30 nm by dual vapor deposition at a vapor deposition rate that satisfies a vapor deposition rate ratio of Compound (3-125)/ETM-1 = 50/50. As the electron injection layer 7 on the electron transport layer 6, lithium fluoride was formed in a film thickness of 1 nm. Finally, aluminum was vapor-deposited in a thickness of 100 nm to form the cathode 8. The characteristics of the organic EL device were measured in the atmosphere at ordinary temperature. Table 1 summarizes the results of measurement of emission characteristics when applying a DC voltage to the fabricated organic EL device.

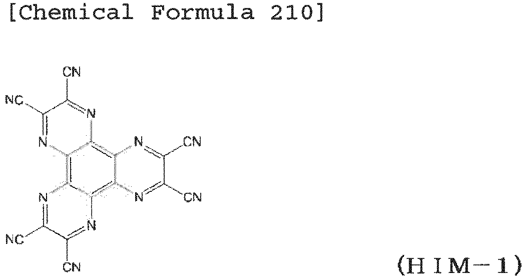

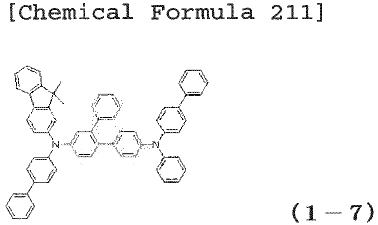

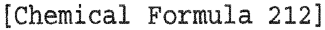

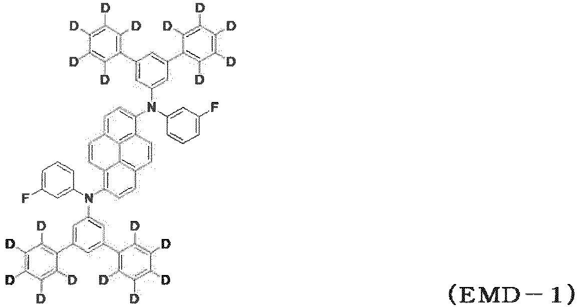

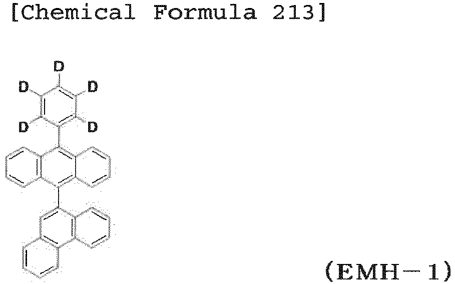

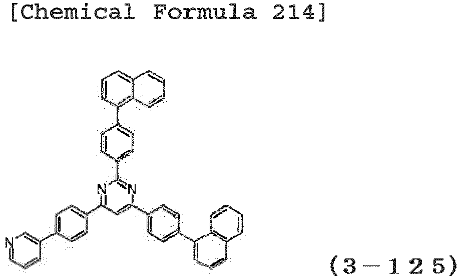

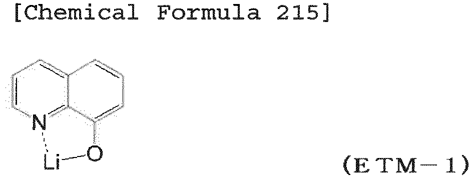

### Example 15

An organic EL device was fabricated under the same conditions used in Example 14, except that Amine derivative (2-1) having a condensed ring structure was used as the material of the light emitting layer 5 instead of Compound (EMD-1) of the above structural formula, and Amine derivative (2-1) having a condensed ring structure and Compound (EMH-1) of the above structural formula were formed in a film thickness of 20 nm by dual vapor deposition at a vapor deposition rate ratio of Amine derivative (2-1)/EMH-1 = 5/95. The characteristics of the thus fabricated organic EL device were measured in the atmosphere at an ordinary temperature. Table 1 summarizes the results of the measurement of emission characteristics performed by applying a direct current voltage to the fabricated organic EL device.

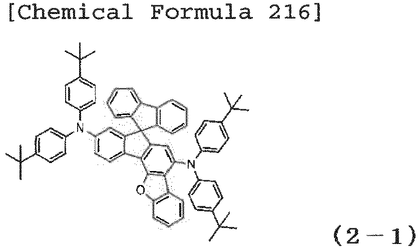

### Comparative Example 1

For comparison, an organic EL device was fabricated under the same conditions used in Example 14, except that the hole transport layer 4 was formed by forming Compound (HTM-1) of the structural formula below in a film thickness of 65 nm, instead of using Compound (1-7) of Example 5. The characteristics of the organic EL device thus fabricated were measured in the atmosphere at an ordinary temperature. Table 1 summarizes the results of emission characteristics measurements performed by applying a direct current voltage to the fabricated organic EL device.

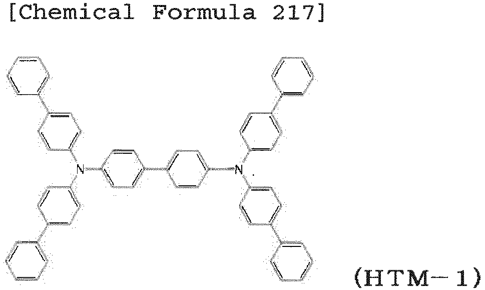

### Comparative Example 2

For comparison, an organic EL device was fabricated under the same conditions used in Example 15, except that the hole transport layer 4 was formed by forming Compound (HTM-1) of the above structural formula in a film thickness of 65 nm, instead of using Compound (1-7) of Example 5. The characteristics of the organic EL device thus fabricated were measured in the atmosphere at an ordinary temperature. Table 1 summarizes the results of emission characteristics measurements performed by applying a direct current voltage to the fabricated organic EL device.

Table 1 summarizes the results of measurement of a device lifetime using the organic EL devices fabricated in Examples 14 to 15 and Comparative Examples 1 to 2. The device lifetime was measured as a time elapsed until the emission luminance of 2,000 cd/m² (initial luminance) at the start of emission was attenuated to 1,900 cd/m² (corresponding to 95% when taking the initial luminance as 100%: Attenuation to 95%) when carrying out constant current driving.

**[Table 1]**

| | Hole transport layer | Light emitting layer | Electron transport layer | voltage [V] (^{®}10mA/cm²) | Luminance [cd/m²] (@10mA/cm²) | Luminous efficiency [cd/A] (@10mA/cm²) | Power efficiency [lm/W] (@10mA/cm²) | Lifetime of device, attenulation to 95% |
|---|---|---|---|---|---|---|---|---|
| Example 14 | 1-7 | EMD-1/ EMH-1 | 3-125/ ETM-1 | 3.96 | 798 | 7.98 | 6.33 | 91 hours |
| Example 15 | 1-7 | 2-1/ EMH-1 | 3-125/ ETM-1 | 3.96 | 854 | 8.55 | 6.78 | 150 hours |
| Comparative Example 1 | HTM-1 | EMD-1/ EMH-1 | 3-125/ ETM-1 | 3.85 | 690 | 6.89 | 5.62 | 66 hours |
| Comparative Example 2 | HTM-1 | 2-1/ EMH-1 | 3-125/ ETM-1 | 3.89 | 760 | 7.60 | 6.14 | 87 hours |

As shown in Table 1, in the comparison of Example 14 and Comparative Example 1 having the same combination of materials of the light emitting layer, the luminance upon passing an electric current with a current density of 10 mA/cm² was 798 cd/m² for the organic EL device in Example 14, which was higher than 690 cd/m² for the organic EL devices in Comparative Example 1. The luminous efficiency upon passing a current with a current density of 10 mA/cm² was 7.98 cd/A for the organic EL devices in Example 14, which was higher than 6.89 cd/A for the organic EL devices in Comparative Example 1. Further, the power efficiency was 6.33 lm/W for the organic EL devices in Example 14, which was higher than 5.62 lm/W for the organic EL devices in Comparative Example 1. The device lifetime (95% attenuation) was 91 hours for the organic EL devices in Example 14, showing achievement of a far longer lifetime than 66 hours for the organic EL device in Comparative Example 2.

Similarly, in the comparison of Example 15 and Comparative Example 2 having the same combination of materials of the light emitting layer, the luminance upon passing an electric current with a current density of 10 mA/cm² was 854 cd/m² for the organic EL device in Example 15, which was higher than 760 cd/m² for the organic EL devices in Comparative Example 2. The luminous efficiency upon passing a current with a current density of 10 mA/cm² was 8.55 cd/A for the organic EL devices in Example 15, which was higher than 7.60 cd/A for the organic EL devices in Comparative Example 2. Further, the power efficiency was 6.78 lm/W for the organic EL devices in Example 15, which was higher than 6.14 lm/W for the organic EL devices in Comparative Example 2. The device lifetime (95% attenuation) was 150 hours for the organic EL devices in Example 15, showing achievement of a far longer lifetime than 87 hours for the organic EL device in Comparative Example 2.

It was found that the organic EL device of the present invention can achieve an organic EL device having high luminous efficiency and a long lifetime compared to the conventional organic EL devices by combining an arylamine compound having a specific structure and an amine derivative having a specific condensed ring structure (and a compound having a specific pyrimidine ring structure) so that carrier balance inside the organic EL device is improved, and further by combining the compounds so that the carrier balance matches the characteristics of the light-emitting material.

### Industrial Applicability

In the organic EL device of the present invention in which an arylamine compound having a specific structure and an amine derivative having a specific condensed ring structure (and a compound having a specific pyrimidine ring structure) are combined, luminous efficiency can be improved, and also durability of the organic EL device can be improved to attain potential applications for, for example, home electric appliances and illuminations.

- 1: Glass substrate
- 2: Transparent anode
- 3: Hole injection layer
- 4: Hole transport layer
- 5: Light emitting layer
- 6: Electron transport layer
- 7: Electron injection layer
- 8: Cathode

## Claims

1. An organic electroluminescent device comprising at least an anode (2),
a hole transport layer (4), a light emitting layer (5),
an electron transport layer (6), and a cathode (8)
in this order, wherein the hole transport layer comprises an arylamine compound of the following general formula (1): 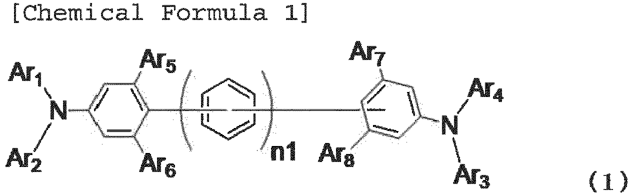
wherein Ar₁ to Ar₄ may be the same or different, and represent a substituted or unsubstituted aromatic hydrocarbon group, a substituted or unsubstituted aromatic heterocyclic group, or a substituted or unsubstituted condensed polycyclic aromatic group;Ar₅ represents a substituted or unsubstituted aromatic hydrocarbon group or a substituted or unsubstituted condensed polycyclic aromatic group; Ar₆ represents a hydrogen atom, a substituted or unsubstituted aromatic hydrocarbon group or a substituted or unsubstituted condensed polycyclic aromatic group,
wherein the substituents in the substituted aromatic hydrocarbon group, or the substituted condensed polycyclic aromatic group of Ar₅ and Ar₆ are selected from the group consisting of a deuterium atom, cyano, nitro, halogen atoms, linear or branched alkyls of 1 to 6 carbon atoms, linear or branched alkyloxys of 1 to 6 carbon atoms, alkenyls, aryloxys, arylalkyloxys, aromatic hydrocarbon groups, condensed polycyclic aromatic groups, arylvinyls and acyls;
Ar₇ and Ar₈ represent a hydrogen atom; and n1 represents 0, 1 or 2, where Ar₃ and Ar₄ may bind to each other to form a ring via a linking group, such as a single bond, substituted or unsubstituted methylene, an oxygen atom, a sulfur atom, or a monosubstituted amino group; and Ar₃ and Ar₄ may bind to the benzene ring binding with -NAr₃Ar₄ group to form a ring via a linking group such as a single bond, substituted or unsubstituted methylene, an oxygen atom, a sulfur atom, or a monosubstituted amino group,
**characterized in that**
the electron transport layer includes a compound of the following general formula (3) having a pyrimidine ring structure: 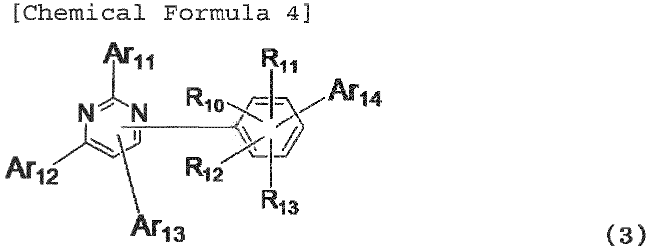
wherein Ar₁₁ represents a substituted or unsubstituted aromatic hydrocarbon group, or a substituted or unsubstituted condensed polycyclic aromatic group, wherein the substituents in the substituted aromatic hydrocarbon group, or the substituted condensed polycyclic aromatic group of Ar₁₁ are selected from the group consisting of a deuterium atom, cyano, nitro, a fluorine atom, a chlorine atom, a bromine atom, an iodine atom, methyl, ethyl, n-propyl, isopropyl, n-butyl, isobutyl, tert-butyl, n-pentyl, isopentyl, neopentyl, n-hexyl, methyloxy, ethyloxy, propyloxy, vinyl, allyl, phenyloxy, tolyloxy, benzyloxy, phenethyloxy, phenyl, biphenylyl, terphenylyl, naphthyl, phenanthrenyl, fluorenyl, indenyl, pyrenyl, perylenyl, fluoranthenyl, triphenylenyl, pyridyl, pyrimidinyl, triazinyl, thienyl, furyl, pyrrolyl, quinolyl, isoquinolyl, benzofuranyl, benzothienyl, indolyl, carbazolyl, benzoxazolyl, benzothiazolyl, quinoxalinyl, benzoimidazolyl, pyrazolyl, dibenzofuranyl, dibenzothienyl, carbolinyl, styryl, naphthylvinyl, acetyl and benzoyl;
Ar₁₂ and Ar₁₃ may be the same or different, and represent a hydrogen atom, a substituted or unsubstituted aromatic hydrocarbon group, or a substituted or unsubstituted condensed polycyclic aromatic group; Ar₁₄ represents a substituted or unsubstituted aromatic heterocyclic group; and R₁₀ to R₁₃ may be the same or different, and represent a hydrogen atom, a deuterium atom, a fluorine atom, a chlorine atom, cyano, trifluoromethyl, linear or branched alkyl of 1 to 6 carbon atoms, a substituted or unsubstituted aromatic hydrocarbon group, a substituted or unsubstituted aromatic heterocyclic group, or a substituted or unsubstituted condensed polycyclic aromatic group, where Ar₁₂ and Ar₁₃ are not simultaneously a hydrogen atom.

2. The organic electroluminescent device according to claim 1, wherein the arylamine compound is an arylamine compound of the following general formula (1a) : 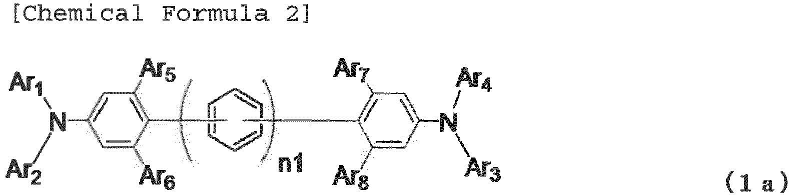
wherein Ar₁ to Ar₄ may be the same or different, and represent a substituted or unsubstituted aromatic hydrocarbon group, a substituted or unsubstituted aromatic heterocyclic group, or a substituted or unsubstituted condensed polycyclic aromatic group; Ar₅ represents a substituted or unsubstituted aromatic hydrocarbon group or a substituted or unsubstituted condensed polycyclic aromatic group; Ar₆ represents a hydrogen atom, a substituted or unsubstituted aromatic hydrocarbon group or a substituted or unsubstituted condensed polycyclic aromatic group,
wherein the substituents in the substituted aromatic hydrocarbon group, or the substituted condensed polycyclic aromatic group of Ar₅ and Ar₆ are selected from the group consisting of a deuterium atom, cyano, nitro, halogen atoms, linear or branched alkyls of 1 to 6 carbon atoms, linear or branched alkyloxys of 1 to 6 carbon atoms, alkenyls, aryloxys, arylalkyloxys, aromatic hydrocarbon groups, condensed polycyclic aromatic groups, arylvinyls and acyls;
Ar₇ and Ar₈ represent a hydrogen atom; and n1 represents 0, 1 or 2, where Ar₃ and Ar₄ may bind to each other to form a ring via a linking group, such as a single bond, substituted or unsubstituted methylene, an oxygen atom, a sulfur atom, or a monosubstituted amino group; and Ar₃ and Ar₄ may bind to the benzene ring binding with -NAr₃Ar₄ group to form a ring via a linking group such as a single bond, substituted or unsubstituted methylene, an oxygen atom, a sulfur atom, or a monosubstituted amino group.

3. The organic electroluminescent device according to claim 1, wherein the light emitting layer includes a blue light emitting dopant.

4. The organic electroluminescent device according to claim 3, wherein the blue light emitting dopant is a pyrene derivative.

5. The organic electroluminescent device according to claim 3, wherein the blue light emitting dopant is an amine derivative having a condensed ring structure of the following general formula (2): 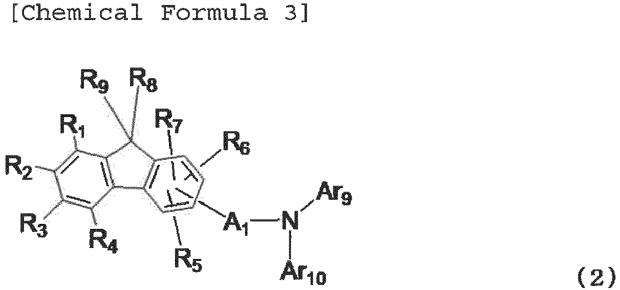 wherein A₁ represents a divalent group of a substituted or unsubstituted aromatic hydrocarbon, a divalent group of a substituted or unsubstituted aromatic heterocyclic ring, a divalent group of substituted or unsubstituted condensed polycyclic aromatics, or a single bond; Arg and Ar₁₀ may be the same or different, and represent a substituted or unsubstituted aromatic hydrocarbon group, a substituted or unsubstituted aromatic heterocyclic group, or a substituted or unsubstituted condensed polycyclic aromatic group, where Ar₉ and Ar₁₀ may bind to each other to form a ring via a linking group, such as a single bond, substituted or unsubstituted methylene, an oxygen atom, a sulfur atom, or a monosubstituted amino group; R₁ to R₄ may be the same or different, and represent a hydrogen atom, a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, substituted or unsubstituted aryloxy, or a disubstituted amino group substituted with a group selected from an aromatic hydrocarbon group, an aromatic heterocyclic group, and a condensed polycyclic aromatic group, where the respective groups may bind to each other to form a ring via a linking group, such as a single bond, substituted or unsubstituted methylene, an oxygen atom, a sulfur atom, or a monosubstituted amino group, and the respective groups bind to the benzene ring binding with R₁ to R₄ to form a ring via a linking group such as substituted or unsubstituted methylene, an oxygen atom, a sulfur atom, or a monosubstituted amino group; R₅ to R₇ may be the same or different, and represent a hydrogen atom, a deuterium atom, a fluorine atom, a chlorine atom, cyano, nitro, linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, linear or branched alkyloxy of 1 to 6 carbon atoms that may have a substituent, cycloalkyloxy of 5 to 10 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy, where the respective groups may bind to each other to form a ring via a linking group, such as a single bond, substituted or unsubstituted methylene, an oxygen atom, a sulfur atom, or a monosubstituted amino group, and the respective groups may bind to the benzene ring binding with R₅ to R₇ to form a ring via a linking group such as substituted or unsubstituted methylene, an oxygen atom, a sulfur atom, or a monosubstituted amino group; and R₈ and R₉ may be the same or different, and represent linear or branched alkyl of 1 to 6 carbon atoms that may have a substituent, cycloalkyl of 5 to 10 carbon atoms that may have a substituent, linear or branched alkenyl of 2 to 6 carbon atoms that may have a substituent, a substituted or unsubstituted aromatic hydrocarbon group, a substituted or unsubstituted aromatic heterocyclic group, a substituted or unsubstituted condensed polycyclic aromatic group, or substituted or unsubstituted aryloxy, where R₈ and R₉ may bind to each other to form a ring via a linking group, such as a single bond, substituted or unsubstituted methylene, an oxygen atom, a sulfur atom, or a monosubstituted amino group.

6. The organic electroluminescent device according to any one of claims 1 to 5, wherein the light emitting layer includes an anthracene derivative.

7. The organic electroluminescent device according to claim 6, wherein the light emitting layer includes a host material which is the anthracene derivative.

## Patentansprüche

1. Organisches elektrolumineszentes Element, umfassend in dieser Reihenfolge mindestens eine Anode (2), eine Lochtransportschicht (4), eine lichtemittierende Schicht (5), eine Elektronentransportschicht (6) und eine Kathode (8),
wobei die Lochtransportschicht eine Arylamin-Verbindung der folgenden allgemeinen Formel (1) umfasst: 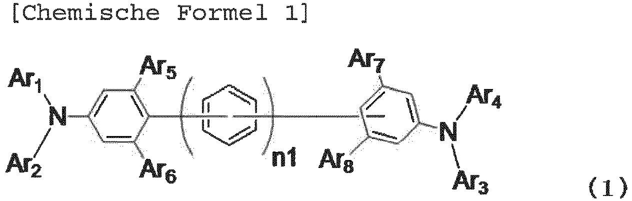
wobei Ar₁ bis Ar₄ gleich oder verschieden sein können und eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellen; Ar₅ eine substituierte oder unsubstituierte aromatische Kohlenwasserstoffgruppe oder eine substituierte oder unsubstituierte kondensierte polycyclische aromatische Gruppe darstellt; Ar₆ ein Wasserstoffatom, eine substituierte oder unsubstituierte aromatische Kohlenwasserstoffgruppe oder eine substituierte oder unsubstituierte kondensierte polycyclische aromatische Gruppe darstellt,
wobei die Substituenten in der substituierten aromatischen Kohlenwasserstoffgruppe oder der substituierten kondensierten polycyclischen aromatischen Gruppe von Ar₅ und Ar₆ ausgewählt sind aus der Gruppe, bestehend aus einem Deuteriumatom, Cyano, Nitro, Halogenatomen, linearen oder verzweigten Alkylen mit 1 bis 6 Kohlenstoffatomen, linearen oder verzweigten Alkyloxylen mit 1 bis 6 Kohlenstoffatomen, Alkenylen, Aryloxylen, Arylalkyloxylen, aromatischen Kohlenwasserstoffgruppen, kondensierten polycyclischen aromatischen Gruppen, Arylvinylen und Acylen;
Ar₇ und Ar₈ ein Wasserstoffatom darstellen; und n1 0, 1 oder 2 darstellt, wobei Ar₃ und Ar₄ aneinander binden können, um einen Ring über eine verbindende Gruppe, wie eine Einfachbindung, substituiertes oder unsubstituiertes Methylen, ein Sauerstoffatom, ein Schwefelatom oder eine einfach substituierte Aminogruppe, zu bilden;
und Ar₃ und Ar₄ an den mit der -NAr₃Ar₄-Gruppe bindenden Benzolring binden können, um einen Ring über eine verbindende Gruppe, wie eine Einfachbindung, substituiertes oder unsubstituiertes Methylen, ein Sauerstoffatom, ein Schwefelatom oder eine einfach substituierte Aminogruppe, zu bilden,
**dadurch gekennzeichnet, dass** die Elektronentransportschicht eine Verbindung der folgenden allgemeinen Formel (3) mit einer Pyrimidinring-Struktur beinhaltet: 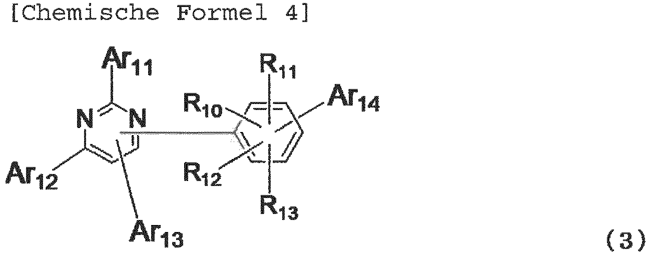
wobei Ar₁₁ eine substituierte oder unsubstituierte aromatische Kohlenwasserstoffgruppe oder eine substituierte oder unsubstituierte kondensierte polycyclische aromatische Gruppe darstellt, wobei die Substituenten in der substituierten aromatischen Kohlenwasserstoffgruppe oder der substituierten kondensierten polycyclischen aromatischen Gruppe von Ar₁₁ ausgewählt sind aus der Gruppe, bestehend aus einem Deuteriumatom, Cyano, Nitro, einem Fluoratom, einem Chloratom, einem Bromatom, einem Iodatom, Methyl, Ethyl, n-Propyl, Isopropyl, n-Butyl, Isobutyl, tert-Butyl, n-Pentyl, Isopentyl, Neopentyl, n-Hexyl, Methyloxy, Ethyloxy, Propyloxy, Vinyl, Allyl, Phenyloxy, Tolyloxy, Benzyloxy, Phenethyloxy, Phenyl, Biphenylyl, Terphenylyl, Naphthyl, Phenanthrenyl, Fluorenyl, Indenyl, Pyrenyl, Perylenyl, Fluoranthenyl, Triphenylenyl, Pyridyl, Pyrimidinyl, Triazinyl, Thienyl, Furyl, Pyrrolyl, Chinolyl, Isochinolyl, Benzofuranyl, Benzothienyl, Indolyl, Carbazolyl, Benzoxazolyl, Benzothiazolyl, Chinoxalinyl, Benzoimidazolyl, Pyrazolyl, Dibenzofuranyl, Dibenzothienyl, Carbolinyl, Styryl, Naphthylvinyl, Acetyl und Benzoyl;
Ar₁₂ und Ar₁₃ gleich oder verschieden sein können und ein Wasserstoffatom, eine substituierte oder unsubstituierte aromatische Kohlenwasserstoffgruppe oder eine substituierte oder unsubstituierte kondensierte polycyclische aromatische Gruppe darstellen; Ar₁₄ eine substituierte oder unsubstituierte aromatische heterocyclische Gruppe darstellt; und R₁₀ bis R₁₃ gleich oder verschieden sein können und ein Wasserstoffatom, ein Deuteriumatom, ein Fluoratom, ein Chloratom, Cyano, Trifluormethyl, lineares oder verzweigtes Alkyl mit 1 bis 6 Kohlenstoffatomen, eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellen, wobei Ar₁₂ und Ar₁₃ nicht gleichzeitig ein Wasserstoffatom sind.

2. Organisches elektrolumineszentes Element nach Anspruch 1, wobei die Arylamin-Verbindung eine Arylamin-Verbindung der folgenden allgemeinen Formel (1a) ist: 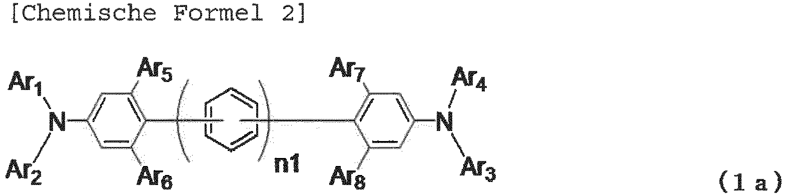
wobei Ar₁ bis Ar₄ gleich oder verschieden sein können und eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellen; Ar₅ eine substituierte oder unsubstituierte aromatische Kohlenwasserstoffgruppe oder eine substituierte oder unsubstituierte kondensierte polycyclische aromatische Gruppe darstellt; Ar₆ ein Wasserstoffatom, eine substituierte oder unsubstituierte aromatische Kohlenwasserstoffgruppe oder eine substituierte oder unsubstituierte kondensierte polycyclische aromatische Gruppe darstellt,
wobei die Substituenten in der substituierten aromatischen Kohlenwasserstoffgruppe oder der substituierten kondensierten polycyclischen aromatischen Gruppe von Ar₅ und Ar₆ ausgewählt sind aus der Gruppe, bestehend aus einem Deuteriumatom, Cyano, Nitro, Halogenatomen, linearen oder verzweigten Alkylen mit 1 bis 6 Kohlenstoffatomen, linearen oder verzweigten Alkyloxylen mit 1 bis 6 Kohlenstoffatomen, Alkenylen, Aryloxylen, Arylalkyloxylen, aromatischen Kohlenwasserstoffgruppen, kondensierten polycyclischen aromatischen Gruppen, Arylvinylen und Acylen;
Ar₇ und Ar₈ ein Wasserstoffatom darstellen; und n1 0, 1 oder 2 darstellt, wobei Ar₃ und Ar₄ aneinander binden können, um einen Ring über eine verbindende Gruppe, wie eine Einfachbindung, substituiertes oder unsubstituiertes Methylen, ein Sauerstoffatom, ein Schwefelatom oder eine einfach substituierte Aminogruppe, zu bilden;
und Ar₃ und Ar₄ an den mit -NAr₃Ar₄ bindenden Benzolring binden können, um einen Ring über eine verbindende Gruppe, wie eine Einfachbindung, substituiertes oder unsubstituiertes Methylen, ein Sauerstoffatom, ein Schwefelatom oder eine einfach substituierte Aminogruppe, zu bilden.

3. Organisches elektrolumineszentes Element nach Anspruch 1, wobei die lichtemittierende Schicht einen blaues Licht emittierenden Dotierstoff enthält.

4. Organisches elektrolumineszentes Element nach Anspruch 3, wobei der blaues Licht emittierende Dotierstoff ein Pyrenderivat ist.

5. Organisches elektrolumineszentes Element nach Anspruch 3, wobei der blaues Licht emittierende Dotierstoff ein Aminderivat mit einer kondensierten Ringstruktur der folgenden allgemeinen Formel (2) ist: 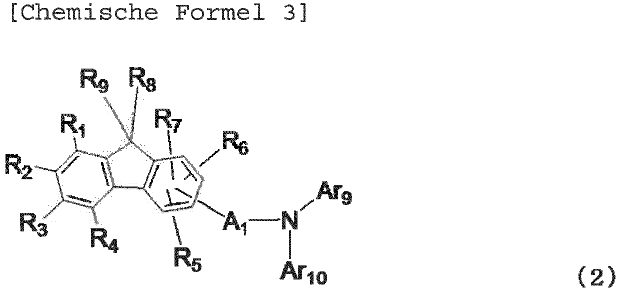 wobei Ar₁ eine zweiwertige Gruppe eines substituierten oder unsubstituierten aromatischen Kohlenwasserstoffs, eine zweiwertige Gruppe eines substituierten oder unsubstituierten aromatischen heterocyclischen Rings, eine zweiwertige Gruppe substituierter oder unsubstituierter kondensierter polycyclischer Aromaten oder eine Einfachbindung darstellt; Ar₉ and Ar₁₀ gleich oder verschieden sein können und eine substituierte oder unsubstituierte aromatische Kohlenwasserstoffgruppe, eine substituierte oder unsubstituierte aromatische heterocyclische Gruppe oder eine substituierte oder unsubstituierte kondensierte polycyclische aromatische Gruppe darstellen, wobei Ar₉ und Ar₁₀ aneinander binden können, um einen Ring über eine verbindende Gruppe, wie eine Einfachbindung, substituiertes oder unsubstituiertes Methylen, ein Sauerstoffatom, ein Schwefelatom oder eine einfach substituierte Aminogruppe, zu bilden; R₁ bis R₄ gleich oder verschieden sein können und ein Wasserstoffatom, ein Deuteriumatom, ein Fluoratom, ein Chloratom, Cyano, Nitro, lineares oder verzweigtes Alkyl mit 1 bis 6 Kohlenstoffatomen, welches einen Substituenten haben kann, Cycloalkyl mit 5 bis 10 Kohlenstoffatomen, welches einen Substituenten haben kann, lineares oder verzweigtes Alkenyl mit 2 bis 6 Kohlenstoffatomen, welches einen Substituenten haben kann, lineares oder verzweigtes Alkyloxy mit 1 bis 6 Kohlenstoffatomen, welches einen Substituenten haben kann, Cycloalkyloxy mit 5 bis 10 Kohlenstoffatomen, welches einen Substituenten haben kann, eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe, substituiertes oder unsubstituiertes Aryloxy oder eine zweifach substituierte Aminogruppe, welche mit einer Gruppe, ausgewählt aus einer aromatischen Kohlenwasserstoffgruppe, einer aromatischen heterocyclischen Gruppe und einer kondensierten polycyclischen aromatischen Gruppe, substituiert ist, darstellen, wobei die jeweiligen Gruppen aneinander binden können, um einen Ring über eine verbindende Gruppe, wie eine Einfachbindung, substituiertes oder unsubstituiertes Methylen, ein Sauerstoffatom, ein Schwefelatom oder eine einfach substituierte Aminogruppe, zu bilden, und die jeweiligen Gruppen an den mit R₁ bis R₄ bindenden Benzolring binden können, um einen Ring über eine verbindende Gruppe, wie substituiertes oder unsubstituiertes Methylen, ein Sauerstoffatom, ein Schwefelatom oder eine einfach substituierte Aminogruppe, zu bilden; R₅ bis R₇ gleich oder verschieden sein können und ein Wasserstoffatom, ein Deuteriumatom, ein Fluoratom, ein Chloratom, Cyano, Nitro, lineares oder verzweigtes Alkyl mit 1 bis 6 Kohlenstoffatomen, welches einen Substituenten haben kann, Cycloalkyl mit 5 bis 10 Kohlenstoffatomen, welches einen Substituenten haben kann, lineares oder verzweigtes Alkenyl mit 2 bis 6 Kohlenstoffatomen, welches einen Substituenten haben kann, lineares oder verzweigtes Alkyloxy mit 1 bis 6 Kohlenstoffatomen, welches einen Substituenten haben kann, Cycloalkyloxy mit 5 bis 10 Kohlenstoffatomen, welches einen Substituenten haben kann, eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder substituiertes oder unsubstituiertes Aryloxy darstellen, wobei die jeweiligen Gruppen aneinander binden können, um einen Ring über eine verbindende Gruppe, wie eine Einfachbindung, substituiertes oder unsubstituiertes Methylen, ein Sauerstoffatom, ein Schwefelatom oder eine einfach substituierte Aminogruppe, zu bilden, und die jeweiligen Gruppen an den mit R₅ bis R₇ bindenden Benzolring binden können, um einen Ring über eine verbindende Gruppe, wie substituiertes oder unsubstituiertes Methylen, ein Sauerstoffatom, ein Schwefelatom oder eine einfach substituierte Aminogruppe, zu bilden; R₈ und R₉ gleich oder verschieden sein können und lineares oder verzweigtes Alkyl mit 1 bis 6 Kohlenstoffatomen, welches einen Substituenten haben kann, Cycloalkyl mit 5 bis 10 Kohlenstoffatomen, welches einen Substituenten haben kann, lineares oder verzweigtes Alkenyl mit 2 bis 6 Kohlenstoffatomen, welches einen Substituenten haben kann, eine substituierte oder unsubstituierte aromatische Kohlenwasserstoffgruppe, eine substituierte oder unsubstituierte aromatische heterocyclische Gruppe, eine substituierte oder unsubstituierte kondensierte polycyclische aromatische Gruppe oder substituiertes oder unsubstituiertes Aryloxy darstellen, wobei R₈ und R₉ aneinander binden können, um einen Ring über eine verbindende Gruppe, wie eine Einfachbindung, substituiertes oder unsubstituiertes Methylen, ein Sauerstoffatom, ein Schwefelatom oder eine einfach substituierte Aminogruppe, zu bilden.

6. Organisches elektrolumineszentes Element nach einem der Ansprüche 1 bis 5, wobei die lichtemittierende Schicht ein Anthracenderivat enthält.

7. Organisches elektrolumineszentes Element nach Anspruch 6, wobei die lichtemittierende Schicht ein Wirtmaterial enthält, welches das Anthracenderivat ist.

## Revendications

1. Dispositif organique électroluminescent comprenant au moins une anode (2), une couche de transport de trous (4), une couche émettrice de lumière (5), une couche de transport d'électrons (6) et une cathode (8) en cet ordre, dans lequel la couche de transport de trous comprend un composé arylamine de la formule générale (1) : 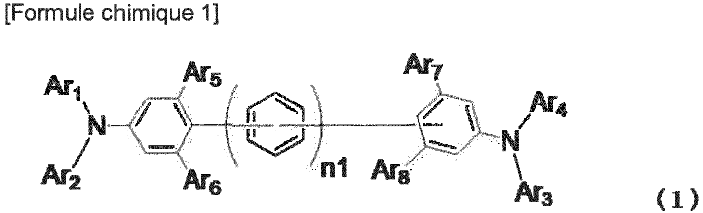
dans laquelle Ar₁ à Ar₄ peuvent être identiques ou différents, et représentent un groupe hydrocarboné aromatique substitué ou non substitué, un groupe hétérocyclique aromatique substitué ou non substitué, ou un groupe aromatique polycyclique condensé substitué ou non substitué ; Ar₅ représente un groupe hydrocarboné aromatique substitué ou non substitué ou un groupe aromatique polycyclique condensé substitué ou non substitué ; Ar₆ représente un atome d'hydrogène, un groupe hydrocarboné aromatique substitué ou non substitué ou un groupe aromatique polycyclique condensé substitué ou non substitué,
dans laquelle les substituants dans le groupe hydrocarboné aromatique substitué ou le groupe aromatique polycyclique condensé substitué de Ar₅ et de Ar₆ sont choisis parmi le groupe consistant en un atome de deutérium, cyano, nitro, les atomes d'halogène, les groupes alkyle linéaire ou ramifié ayant 1 à 6 atomes de carbone, les groupes alcoxy linéaire ou ramifié ayant 1 à 6 atomes de carbone, les groupes alcényle, les groupes aryloxy, les groupes arylalkyloxy, les groupes hydrocarbonés aromatiques, les groupes aromatiques polycycliques condensés, les groupes arylvinyle ou les groupes acyle ;
Ar₇ et Ar₈ représentent un atome d'hydrogène ; et n1 représente 0, 1 ou 2,
dans laquelle Ar₃ et Ar₄ peuvent être liés l'un à l'autre pour former un cycle via un groupe de liaison, comme une simple liaison, un méthylène substitué ou non substitué, un atome d'oxygène, un atome de soufre, ou un groupe amino monosubstitué ; et Ar₃ et Ar₄ peuvent être liés au cycle benzénique lié au groupe -NAr₃Ar₄ pour former un cycle via un groupe de liaison, comme une simple liaison, un méthylène substitué ou non substitué, un atome d'oxygène, un atome de soufre, ou un groupe amino monosubstitué,
**caractérisé en ce que** la couche de transport d'électrons comprend un composé ayant une structure à cycle pyrimidine, de la formule générale (3) suivante : 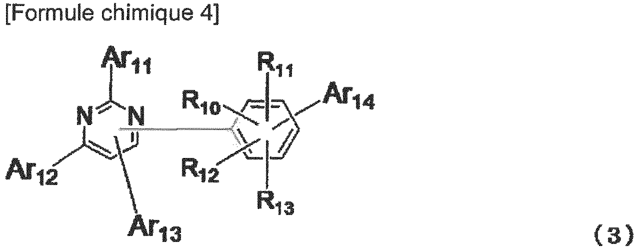
dans laquelle Ar₁₁ représente un groupe hydrocarboné aromatique substitué ou non substitué, ou un groupe aromatique polycyclique condensé substitué ou non substitué, dans laquelle les substituants dans le groupe hydrocarboné aromatique substitué ou le groupe aromatique polycyclique condensé substitué de Ar₁₁ sont choisis parmi le groupe consistant en un atome de deutérium, cyano, nitro, un atome de fluor, un atome de chlore, un atome de brome, un atome d'iode, méthyle, éthyle, n-propyle, isopropyle, n-butyle, isobutyle, t-butyle, n-pentyle, isopentyle, néopentyle, n-hexyle, méthyloxy, éthyloxy, propyloxy, vinyle, allyle, phényloxy, tolyloxy, benzyloxy, phénéthyloxy, phényle, biphénylyle, terphénylyle, naphtyle, phénanthrényle, fluorényle, indényle, pyrényle, pérylényle, fluoranthényle, triphénylényle, pyridyle, pyrimidinyle, triazinyle, thiényle, furyle, pyrrolyle, quinolyle, isoquinolyle, benzofuranyle, benzothiényle, indolyle, carbazolyle, benzoxazolyle, benzothiazolyle, quinoxalinyle, benzoimidazolyle, pyrazolyle, dibenzofuranyle, dibenzothiényle, carbolinyle, styryle, naphtylvinyle, acétyle et benzoyle ;
Ar₁₂ et Ar₁₃ peuvent être identiques ou différents et représentent un atome d'hydrogène, un groupe hydrocarboné aromatique substitué ou non substitué, ou un groupe aromatique polycyclique condensé substitué ou non substitué ; Ar₁₄ représente un groupe aromatique hétérocyclique substitué ou non substitué ; et R₁₀ à R₁₃ peuvent être identiques ou différents et représentent un atome d'hydrogène, un atome de deutérium, un atome de fluor, un atome de chlore, cyano, trifluorométhyle, un groupe alkyle linéaire ou ramifié ayant 1 à 6 atomes de carbone, un groupe hydrocarboné aromatique substitué ou non substitué, un groupe aromatique hétérocyclique substitué ou non substitué, ou un groupe aromatique polycyclique condensé substitué ou non substitué, où Ar₁₂ et Ar₁₃ ne sont pas simultanément un atome d'hydrogène.

2. Dispositif organique électroluminescent selon la revendication 1, dans lequel le composé arylamine est un composé arylamine de la formule générale (1a) suivante : 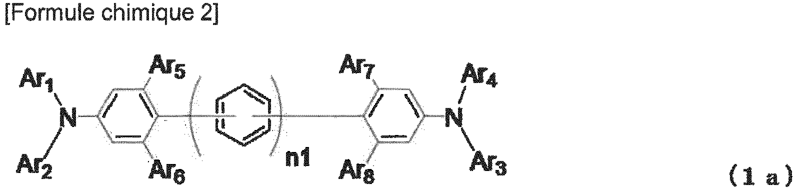
dans laquelle Ar₁ à Ar₄ peuvent être identiques ou différents, et représentent un groupe hydrocarboné aromatique substitué ou non substitué, un groupe hétérocyclique aromatique substitué ou non substitué, ou un groupe aromatique polycyclique condensé substitué ou non substitué ; Ar₅ représente un groupe hydrocarboné aromatique substitué ou non substitué ou un groupe aromatique polycyclique condensé substitué ou non substitué ; Ar₆ représente un atome d'hydrogène, un groupe hydrocarboné aromatique substitué ou non substitué ou un groupe aromatique polycyclique condensé substitué ou non substitué,
dans laquelle les substituants dans le groupe hydrocarboné aromatique substitué ou le groupe aromatique polycyclique condensé substitué de Ar₅ et de Ar₆ sont choisis parmi le groupe consistant en un atome de deutérium, cyano, nitro, les atomes d'halogène, les groupes alkyle linéaire ou ramifié ayant 1 à 6 atomes de carbone, les groupes alcoxy linéaire ou ramifié ayant 1 à 6 atomes de carbone, les groupes alcényle, les groupes aryloxy, les groupes arylalkyloxy, les groupes hydrocarbonés aromatiques, les groupes aromatiques polycycliques condensés, les groupes arylvinyle ou les groupes acyle ;
Ar₇ et Ar₈ représentent un atome d'hydrogène ; et n1 représente 0, 1 ou 2,
dans laquelle Ar₃ et Ar₄ peuvent être liés l'un à l'autre pour former un cycle via un groupe de liaison, comme une simple liaison, un méthylène substitué ou non substitué, un atome d'oxygène, un atome de soufre, ou un groupe amino monosubstitué ; et Ar₃ et Ar₄ peuvent être liés au cycle benzénique lié au groupe -NAr₃Ar₄ pour former un cycle via un groupe de liaison, comme une simple liaison, un méthylène substitué ou non substitué, un atome d'oxygène, un atome de soufre, ou un groupe amino monosubstitué.

3. Dispositif organique électroluminescent selon la revendication 1, dans lequel la couche émettrice de lumière comprend un dopant émettant de la lumière bleue.

4. Dispositif organique électroluminescent selon la revendication 3, dans lequel le dopant émettant de la lumière bleue est un dérivé du pyrène.

5. Dispositif organique électroluminescent selon la revendication 3, dans lequel le dopant émettant de la lumière bleue est un dérivé d'amine ayant un structure cyclique condensée de la formule générale (2) suivante : 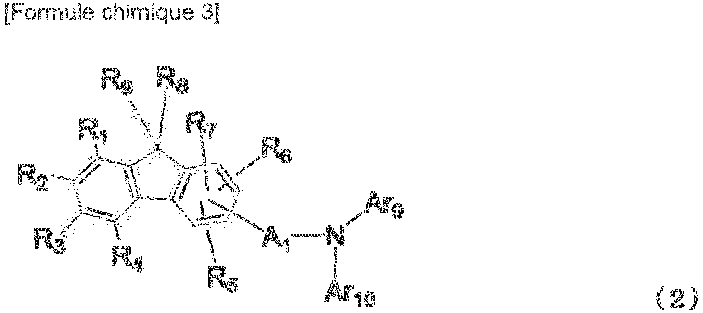 dans laquelle A₁ représente un groupe bivalent d'un hydrocarbure aromatique substitué ou non substitué, un groupe bivalent d'un hétérocycle aromatique substitué ou non substitué, un groupe bivalent d'un aromatique polycyclique condensé substitué ou non substitué, ou une simple liaison ; Ar₉ et Ar₁₀ peuvent être identiques ou différents, et représentent un groupe hydrocarboné aromatique substitué ou non substitué, un groupe aromatique hétérocyclique substitué ou non substitué, ou un groupe aromatique polycyclique condensé substitué ou non substitué, où Ar₉ et Ar₁₀ peuvent être liés l'un à l'autre pour former un cycle via un groupe de liaison, comme une simple liaison, un méthylène substitué ou non substitué, un atome d'oxygène, un atome de soufre, ou un groupe amino monosubstitué ; R₁ à R₄ peuvent être identiques ou différents et représentent un atome d'hydrogène, un atome de deutérium, un atome de fluor, un atome de chlore, cyano, nitro, un groupe alkyle linéaire ou ramifié ayant 1 à 6 atomes de carbone qui peut porter un substituant, un groupe cycloalkyle ayant 5 à 10 atomes de carbone qui peut porter un substituant, un groupe alcényle linéaire ou ramifié ayant 2 à 6 atomes de carbone qui peut porter un substituant, un groupe alkyloxy linéaire ou ramifié ayant 1 à 6 atomes de carbone qui peut porter un substituant, un groupe cycloalkyloxy ayant 5 à 10 atomes de carbone qui peut porter un substituant, un groupe hydrocarboné aromatique substitué ou non substitué, un groupe aromatique hétérocyclique substitué ou non substitué, un groupe aromatique polycyclique condensé substitué ou non substitué, un groupe aryloxy substitué ou non substitué, ou un groupe amino disubstitué substitué par un groupe choisi parmi un groupe hydrocarboné aromatique, un groupe aromatique hétérocyclique, et un groupe aromatique polycyclique condensé, où les groupes respectifs peuvent être liés les uns aux autres pour former un cycle via un groupe de liaison, comme une simple liaison, un méthylène substitué ou non substitué, un atome d'oxygène, un atome de soufre, ou un groupe amino monosubstitué, et les groupes respectifs sont liés au cycle benzénique liant avec R₁ à R₄ pour former un cycle via un groupe de liaison comme un méthylène substitué ou non substitué, un atome d'oxygène, un atome de soufre, ou un groupe amino monosubstitué ; R₅ à R₇ peuvent être identiques ou différents et représentent un atome d'hydrogène, un atome de deutérium, un atome de fluor, un atome de chlore, cyano, nitro, un groupe alkyle linéaire ou ramifié ayant 1 à 6 atomes de carbone qui peut porter un substituant, un groupe cycloalkyle ayant 5 à 10 atomes de carbone qui peut porter un substituant, un groupe alcényle linéaire ou ramifié ayant 2 à 6 atomes de carbone qui peut porter un substituant, un groupe alkyloxy linéaire ou ramifié ayant 1 à 6 atomes de carbone qui peut porter un substituant, un groupe cycloalkyloxy ayant 5 à 10 atomes de carbone qui peut porter un substituant, un groupe hydrocarboné aromatique substitué ou non substitué, un groupe aromatique hétérocyclique substitué ou non substitué, un groupe aromatique polycyclique condensé substitué ou non substitué, ou un groupe aryloxy substitué ou non substitué, où les groupes respectifs peuvent être liés les uns aux autres pour former un cycle via un groupe de liaison, comme une simple liaison, un méthylène substitué ou non substitué, un atome d'oxygène, un atome de soufre, ou un groupe amino monosubstitué, et les groupes respectifs peuvent être liés au cycle benzénique liant avec R₅ à R₇, pour former un cycle via un groupe de liaison comme un méthylène substitué ou non substitué, un atome d'oxygène, un atome de soufre, ou un groupe amino monosubstitué ; et R₈ et R₉ peuvent être identiques ou différents et représentent un groupe alkyle linéaire ou ramifié ayant 1 à 6 atomes de carbone qui peut porter un substituant, un groupe cycloalkyle ayant 5 à 10 atomes de carbone qui peut porter un substituant, un groupe alcényle linéaire ou ramifié ayant 2 à 6 atomes de carbone qui peut porter un substituant, un groupe hydrocarboné aromatique substitué ou non substitué, un groupe aromatique hétérocyclique substitué ou non substitué, un groupe aromatique polycyclique condensé substitué ou non substitué, ou un groupe aryloxy substitué ou non substitué, où R₈ et R₉ peuvent être liés l'un à l'autre pour former un cycle via un groupe de liaison, comme une simple liaison, un méthylène substitué ou non substitué, un atome d'oxygène, un atome de soufre, ou un groupe amino monosubstitué.

6. Dispositif organique électroluminescent selon l'une quelconque des revendications 1 à 5, dans lequel la couche émettrice de lumière comprend un dérivé de l'anthracène.

7. Dispositif organique électroluminescent selon la revendication 6, dans lequel la couche émettrice de lumière comprend un matériau hôte qui est le dérivé de l'anthracène.
